Europäisches Patentamt

European Patent Office

Office européen des brevets

(19)

(11) Veröffentlichungsnummer: **0 464 046 B1**

(12) **EUROPÄISCHE PATENTSCHRIFT**

(45) Veröffentlichungstag der Patentschrift: **20.04.94**

(21) Anmeldenummer: **90904308.5**

(22) Anmeldetag: **13.02.90**

(86) Internationale Anmeldenummer:
**PCT/EP90/00230**

(87) Internationale Veröffentlichungsnummer:
**WO 90/09367 (23.08.90 90/20)**

(51) Int. Cl.5: **C07C 69/82**, C07C 67/52,
C07C 67/39

(54) **VERFAHREN ZUR HERSTELLUNG VON DMT-ZWISCHENPRODUKT BESTIMMTER REINHEIT SOWIE DESSEN WEITERVERARBEITUNG ZU REINST-DMT UND/ODER MITTEL- ODER HOCHREINER TEREPHTHALSÄURE.**

(30) Priorität: **16.02.89 DE 3904586**

(43) Veröffentlichungstag der Anmeldung:
**08.01.92 Patentblatt 92/02**

(45) Bekanntmachung des Hinweises auf die Patenterteilung:
**20.04.94 Patentblatt 94/16**

(84) Benannte Vertragsstaaten:
**AT BE CH DE DK ES FR GB IT LI LU NL SE**

(56) Entgegenhaltungen:
**EP-A- 0 096 082**
**DE-A- 3 011 585**
**FR-A- 1 245 493**
**US-A- 2 646 393**
**US-A- 3 502 711**

(73) Patentinhaber: **HÜLS AKTIENGESELLSCHAFT**

**D-45764 Marl(DE)**

(72) Erfinder: **KORTE, Hermann-Joseph**
**Stahlenstrasse 33 a**
**D-5216 Niederkassel 2(DE)**
Erfinder: **MILETIC, Anton**
**Bertha-v.-Suttner-Str. 9**
**D-5210 Troisdorf(DE)**
Erfinder: **NEUTZLER, Hans, U.**
**Am Knapp 6**
**D-5802 Wetter(DE)**
Erfinder: **SCHOENGEN, Anton**
**In den Espeln 9**
**D-5810 Witten(DE)**
Erfinder: **SCHROEDER, Johann, Heinrich**
**Ortli 5**
**D-4600 Dortmund 30(DE)**
Erfinder: **WIRGES, Ralf**
**Porzer Str. 36**
**D-5216 Niederkassel(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von DMT-Zwischenprodukt gemäß dem Oberbegriff des Anspruchs 1 sowie dessen Weiterverarbeitung zu mittel- oder hochreinem Dimethylterephthalat (DMT) und/oder mittel- oder hochreiner Terephthalsäure (TA).

Dimethylterephthalat (DMT) wird in zahlreichen großtechnischen Anlagen nach dem sogenannten Witten-DMT-Verfahren (vgl. DE-PS 10 41 945) hergestellt. Durch Umsetzung mehrfunktioneller Alkohole mit DMT werden Polyester gewonnen. Solche - auch als gesättigte Polyester bezeichneten - hochmolekularen Verbindungen werden u.a. zu Fasern, Fäden, Filmen oder Formteilen verarbeitet.

### Stand der Technik

Nach dem Witten-DMT-Verfahren wird ein Gemisch aus para-Xylol (p-X) und para-Toluylsäure-methylester (p-TE) in der flüssigen Phase in Abwesenheit von Lösungsmitteln und von Halogenverbindungen bei einem Druck von etwa 4 bis 8 bar und einer Temperatur von etwa 140 bis 180 °C mit Luftsauerstoff in Gegenwart von gelösten Schwermetalloxidationskatalysatoren oxidiert, z.B. in Gegenwart eines Gemisches von Kobalt- und Mangan-Verbindungen (vgl. DE-PS 20 10 137).

Im Anschluß an die Oxidationsstufe wird das erhaltene Reaktionsgemisch, das überwiegend aus Monomethylterephthalat (MMT), p-Toluylsäure (p-TA) und Terephthalsäure (TA), gelöst bzw. suspendiert in para-Toluylsäure-methylester (pT-Ester) und DMT, besteht, mit Methanol bei einem Druck von 20 bis 25 bar und einer Temperatur von etwa 250 bis 280 °C verestert. Höhere Drücke sind technisch möglich, werden aber aus Kostengründen nicht angewandt. Das Veresterungsprodukt wird destillativ aufgetrennt in eine p-TE-Fraktion, eine Roh-DMT-Fraktion und einen hochsiedenden, teerartigen Rückstand. Die p-TE-Fraktion wird in die Oxidation zurückgeführt. Der hochsiedende, teerartige Destillationsrückstand enthält u.a. sämtliche Bestandteile des Katalysatorsystems, die gemäß der EP-PS 0 053 241 wiedergewonnen und in die Oxidation zurückgeführt werden können.

Das aus der Destillation stammende Roh-DMT mit einer typischen Reinheit von 97 bis 99,9 Gew.-% enthält neben ca. 0,05 bis 2 % an Isomeren des DMT (Dimethylortho- und Dimethylisophthalat [DMO, DMI]) noch z.T. störende Mengen an Terephthalaldehydsäure-methylester (TAE), Monomethylterephthalat (MMT), para-Toluylsäure (p-TA) und anderen aus dem eingesetzten para-Xylol oder Nebenreaktionen stammende Verunreinigungen.

Zur Reinigung des Roh-DMT zu faserreinem Reinst-DMT (DMT-p), bei der insbesondere TAE und die Isomeren entfernt werden müssen, ist es bekannt, das Roh-DMT durch methanolische Umkristallisation zu behandeln (DE-OS 20 14 012, Hydrocarbon Processing, Nov. 1983, S. 91). Zur Erzielung einer zur Faserherstellung ausreichenden Reinheit des DMT (Summe der Verunreinigungen einschließlich der Isomeren DMO und DMI kleiner als 100 ppm) war es z. T. üblich, die Umkristallisation 2-fach mit zwischengeschalteter Wäsche mit Methanol durchzuführen, wobei das Methanol im Gegenstrom geführt wurde. Zusätzlich war bislang bei einem Roh-DMT geringer Reinheit noch eine abschließende Destillation des DMT notwendig.

Der bei der methanolischen Umkristallisation aus der Mutterlauge durch Filtrieren gewonnene Filtratrückstand enthält neben den Isomeren DMO und DMI noch große Mengen an DMT und anderen Wertprodukten (Zwischenprodukte bei der DMT-Herstellung), so daß der Filtratrückstand bislang grundsätzlich zum größten Teil in die Oxidation zurückgeführt wurde. Durch diese Filtratrückstands-Rückführung reichern sich die Isomeren bis auf Werte von 8 bis 12 Gew.-% an, so daß die vorgeschalteten Reaktoren und vor allem die Destillation und Umkristallisation zusätzliche Kapazitäten aufweisen müssen. Die Filtratrückstands-Rückführung war beim Stand der Technik auch deshalb notwendig, um genügend "Neutralprodukte" zur Einstellung des Schmelzpunktes und der Säurezahl in der Oxidation zu erhalten sowie die Fließfähigkeit des Oxidationsproduktes zur Veresterung zu gewährleisten.

Aus der DE-PS 30 11 858 und DE-PS 29 16 197 ist bekannt, daß der Anteil an Terephthalaldehydsäure-methylester (TAE) im DMT-Zwischenprodukt insbesondere bei der Weiterverarbeitung zu Terephthalsäure hoher Reinheit (PTA) möglichst gering gehalten werden muß. In der DE-PS 30 11 858 wird zur Verringerung des TAE-Gehaltes im Roh-DMT eine eigene, sehr aufwendige Rektifikationskolonne vorgeschlagen, wobei der TAE-Gehalt im Roh-DMT bis auf Werte von < 0,01 Gew.-% reduziert werden kann.

Trotz dieses hohen Aufwandes hat es sich bei diesem Verfahren herausgestellt, daß unter ungünstigen Bedingungen im Endprodukt Terephthalsäure (TA) ein immer noch störend hoher Anteil an Terephthalaldehydsäure (TAS, 4-CBA), der bei der Hydrolyse aus dem TAE gebildeten Säure, vorliegen kann. Der Restgehalt an TAS in der Terephthalsäure konnte insbesondere dann unerklärlich hohe Werte annehmen, wenn das Roh-DMT vor der Weiterverarbeitung zu Terephthalsäure zwischengelagert werden mußte.

2

## Aufgabe der Erfindung

Aufgabe der vorliegenden Erfindung ist es, das gattungsgemäße Verfahren zur Herstellung von DMT-Zwischenprodukt dahingehend zu verbessern, daß bei der Weiterverarbeitung des DMT-Zwischenproduktes zu Reinst-DMT der TAE-Gehalt und bei der Weiterverarbeitung zu PTA der TAS-Gehalt weiter verringert wird. Insbesondere soll auch bei längerer Zwischenlagerung des Roh-DMT und anschließender Weiterverarbeitung zu Reinst-DMT bzw. PTA der TAE-Gehalt bzw. der TAS-Gehalt im Endprodukt möglichst gering gehalten werden.

Weitere Anliegen der Erfindung sind die Senkung des apparativen und energetischen Aufwandes der DMT- und PTA-Herstellung, die Steigerung der Ausbeute und die Schaffung einer Möglichkeit, in einer Anlage gleichzeitig DMT-p und PTA herzustellen.

## Darstellung der Erfindung

Diese Aufgabe wird gemäß der Erfindung durch die kennzeichnenden Merkmale des Anspruchs 1 gelöst.

Wesentliches Merkmal der Erfindung ist es, die Reinigung des Roh-DMT durch kostengünstige Rektifikation und nachgeschaltete einfache Lösungsmittel-Umkristallisation zu verbessern und zu vereinfachen.

Der größte Teil der Verunreinigungen - mit Ausnahme der Isomeren - wird erfindungsgemäß durch Rektifikation entfernt, so daß das so gereinigte Roh-DMT (Fraktion II) bevorzugt einen TAE- und Hydroxymethyl-benzoesäuremethylester-Gehalt (HM-BME) von zusammen weniger als 0,2 Gew.-% (2.000 ppm) aufweist. Die Rektifikation des Ester-Gemisches kann insbesondere deshalb im Verhältnis zu dem Verfahren gemäß der DE-PS 30 11 858 vereinfacht werden, weil die schwersiedende Rückstandsfraktion III einen hohen DMT-Gehalt von 15 bis 70 Gew.-%, bevorzugt 40 bis 60 Gew.-%, aufweist.

Ebenso kann die p-TE-reiche Fraktion I einen hohen DMT-Anteil aufweisen, bevorzugt liegt dieser bei 20 bis 30 Gew.-%.

Die Rückstandsfraktion III kann z.B. mit einem Verfahren gemäß der DE-PS 24 27 875 aufbereitet werden. Nach einer bevorzugten Ausführung der Erfindung wird die Rückstandsfraktion jedoch durch zweistufige Methanolyse aufbereitet, wobei das DMT sowie weitere Wertprodukte in die Oxidation zurückgeführt werden. Insgesamt beträgt der DMT-Anteil, der aus der Rückstandsfraktion wiedergewonnen und in die Oxidation zurückgeführt wird, bevorzugt 3 bis 7 Gew.-%, bezogen auf die Summe aller der Oxidation in flüssiger Phase zugeführten Komponenten.

Bevorzugt wird die p-TE-Fraktion I im Überschuß in die Oxidation zurückgeführt, so daß im entstehenden Säure-Gemisch noch etwa 5 bis 30 Gew.-% p-TE, insbesondere 20 bis 25 Gew.-%, enthalten sind.

Der eingestellte p-TE-Überschuß sowie die im Kreislauf gefahrenen DMT-Mengen dienen zur Reaktionssteigerung und zur Schmelzpunkteinstellung des den Oxidator verlassenden Produktstromes. Es werden Schmelzpunkte von 130 bis 150 °C bei einer Säurezahl von 200 bis 300 g KOH/kg Oxidat bevorzugt.

Das durch Rektifikation gereinigte Roh-DMT (Fraktion II) wird anschließend einer einfachen Lösungsmittel-Umkristallisation, bevorzugt in Methanol unterworfen, um Restmengen an TAE, HM-BME und anderen Verunreinigungen zu entfernen. Gleichzeitig können in dieser Stufe die Isomeren DMO und DMI entfernt werden.

Nach einer bevorzugten Ausführung der Erfindung wird die aus der (ersten) methanolischen Umkristallisation stammende Mutterlauge durch Abdampfen von Methanol zunächst eingedickt. Die gelösten DMT-Mengen mit dem Lösungsgleichgewicht entsprechenden Mengen an Isomeren und Verunreinigungen werden anschließend durch weitere Abkühlung auf ca. 20 bis 50 °C, bevorzugt ca. 25 °C, auskristallisiert, abgefiltert und nach Vermischen mit Methanol vor die (erste) Umkristallisation zurückgefahren. Die in der verbleibenden Mutterlauge noch gelösten Stoffe, überwiegend DMO, DMI und Reste von DMT sowie die in der Rektifikation nicht entfernten Mengen an TAE und HM-BME werden durch Abdampfen des restlichen Methanols aufkonzentriert und als Rückstand aus dem Prozeß ausgeschleust oder in die Oxidation zurückgeführt.

Soweit, wie nach dem Stand der Technik üblich, das eingesetzte p-Xylol einen Anteil an Isomeren von über 1.000 bis 5.000 ppm aufweist, wird nach einer bevorzugten Ausführung der Erfindung auf eine Rückführung des hierbei anfallenden Rückstandes in die Oxidation vollständig verzichtet. Eine vollständige Ausschleusung des Rückstandes ist nur deshalb nicht mit unwirtschaftlichen Verlusten behaftet, weil in der vorangehenden Rektifikation die Wertprodukte TAE und HM-BME bereits nahezu vollständig aus dem Roh-DMT entfernt werden. Gleichzeitig wird eine Rückstands-Rückführung- im Gegensatz zu den bekannten Verfahren - auch nicht zur Einstellung des Schmelzpunktes und der Säurezahl im Oxidator benötigt, da die hierzu benötigten Neutralprodukt-Mengen als DMT-Anteile der p-TE-Fraktion I und der Rückstandsfraktion III in die Oxidation geleitet werden.

Bei an Isomeren besonders armem p-Xylol, mit Isomeren-Anteilen unter 100 bis 500 ppm, kann ggf. auch der gesamte Rückstand in die Oxidation zurückgeführt werden, soweit ein entsprechender Anteil an Isomeren im Endprodukt toleriert werden kann. Bei diesen geringen Isomeren-Anteilen im p-Xylol, das nach dem jetzigen Stand der Technik allerdings nicht in ausreichenden Mengen zur Verfügung steht, ist die zurückgeführte Rückstands-Menge jedoch verhältnismäßig gering, sie beträgt etwa 3 bis 6 Gew.-% der aus der Rektifikation in die Oxidation zurückgeführten DMT-Menge.

Je nach geforderter Endprodukt-Reinheit und Isomerenanteil im p-Xylol kann ggf. auch ein Teil des Filtratrückstandes in die Oxidation zurückgeführt werden, wobei der so in die Oxidation zurückgeführte Anteil an DMT und dessen Isomeren im Verhältnis zu den DMT-Mengen, die aus der Rektifikation in die Oxidation zurückgeführt werden, gering ist.

Ein weiteres wesentliches Merkmal der Erfindung ist es, neben TAE auch den Anteil der im DMT-Zwischenprodukt vorhandenen Verunreinigung Hydroxymethyl-benzoesäuremethylester (HM-BME) zu minimieren.

Es hat sich überraschend herausgestellt, daß der Anteil an HM-BME im Roh-DMT neben dem Anteil von TAE entscheidend für den TAE-Gehalt im Reinst-DMT bzw. für den Anteil an TAS in PTA ist. Es wird vermutet, daß sich HM-BME in dem sich an die DMT-Herstellung anschließenden Zwischenlagerbehälter bzw. in den nachfolgenden Verfahrensstufen mit Luftsauerstoff zu TAE umsetzt, wobei der Luftsauerstoff durch unvermeidbare Mikro-Leckagen im Destillationsteil, der gegenüber dem atmosphärischen Druck mit Unterdruck gefahren wird, in die geschlossene Anlage gelangen kann. Dieser Zusammenhang war bis zum Zeitpunkt der Erfindung unbekannt.

Durch das erfindungsgemäße Verfahren zur Herstellung von DMT-Zwischenprodukt ist es erstmals möglich, die nachfolgenden Verfahrensschritte zur Weiterverarbeitung zu Reinst-DMT und/oder PTA wesentlich zu vereinfachen, ohne daß der TAE-Gehalt im Reinst-DMT bzw. der TAS-Gehalt in PTA höhere Werte annimmt. Auch bei längerer Zwischenlagerung des DMT-Zwischenproduktes kann zuverlässig garantiert werden, daß der Anteil an TAE bzw. TAS im Endprodukt nicht weiter ansteigt, so daß eine gleichbleibende Qualität erreicht wird.

Das erfindungsgemäße DMT-Zwischenprodukt mit einem typischen Gehalt an Isomeren, TAE und HM-BME von insgesamt 50 bis 200 ppm kann durch eine zweite Umkristallisation in Methanol mit geringem apparativen Aufwand zu Reinst-DMT mit einer typischen Reinheit von > 99,995 Gew.-% weiterverarbeitet werden. Durch eine zusätzliche Wäsche des DMT-Zwischenproduktes vor der abschliessenden Umkristallisation läßt sich der Gehalt an Verunreinigungen bei Bedarf nochmals auf Werte < 10 ppm verringern.

Die Weiterverarbeitung des DMT-Zwischenproduktes zu Terephthalsäure erfolgt bevorzugt durch Hydrolyse in einem einfachen Reaktor, wobei das entstehende Methanol mit dem als Nebenprodukt anfallenden Dimethylether (DME) durch Einleitung von Strippdampf entfernt wird. Die gebildete Terephthalsäure wird, in Wasser suspendiert bzw. gelöst, aus dem Reaktor ausgeschleust und mehrstufig auskristallisiert, zentrifugiert und getrocknet. Soweit das DMT-Zwischenprodukt ohne zusätzliche Wäsche direkt in die Hydrolyse gegeben wird, enthält die gebildete mittelreine Terephthalsäure (MTA) typisch 500 bis 1.000 ppm an Verunreinigungen. Mit einer einfachen Wäsche des DMT-Zwischenproduktes in Methanol und anschließendem Zentrifugieren läßt sich die Reinheit der Terephthalsäure auf 100 bis 150 ppm steigern (PTA), wobei die Verunreinigungen hauptsächlich aus MMT bestehen. Durch einfache Wäsche der PTA in Wasser kann auch der MMT-Gehalt, soweit erforderlich, nochmals eingeschränkt werden, so daß eine Terephthalsäure bislang großtechnisch kaum erreichter Reinheit mit einer Summe aller Verunreinigungen einschließlich MMT von < 50 ppm hergestellt werden kann (PTA-p).

Weiterhin wird mit dem erfindungsgemäßen Verfahren eine wesentliche Einsparung an Energie erzielt. Von besonderem Wert ist schließlich die Möglichkeit, das erfindungsgemäße DMT-Zwischenprodukt sowohl zu Reinst-DMT (DMT-p) als auch zu mittel- und hochreiner Terephthalsäure (MTA bzw. PTA) weiterzuverarbeiten, wobei sich diese Weiterverarbeitung, z.B. im Vergleich zu dem DMT-Zwischenprodukt gemäß der DE-PS 29 16 197 und DE-PS 30 11 858, wesentlich vereinfacht, da das erfindungsgemäße DMT-Zwischenprodukt nur sehr geringe Restgehalte an Isomeren aufweist. Schließlich eröffnet das erfindungsgemäße Verfahren die Möglichkeit, Terephthalsäure höchster Reinheit (PTA-p) mit gegenüber üblicher PTA nochmals verringerten Anteilen an Verunreinigungen, insbesondere auch an MMT und p-TA, herzustellen.

Von besonderem Vorteil ist schließlich die Möglichkeit, mit geringem zusätzlichem apparativem und energetischem Aufwand gleichzeitig Reinst-DMT und mittel- bzw. hochreine und höchstreine Terephthalsäure herzustellen, wobei die produzierten Mengenanteile DMT-p/PTA und PTA-p beliebig verändert werden können.

### Kurze Beschreibung der Zeichnung

Das erfindungsgemäße Verfahren wird nachfolgend anhand von Ausführungsbeispielen sowie der Zeichnung erläutert.

Es zeigen dabei als Anlagenschemen

Fig. 1    eine Anlage zur Herstellung von Roh-DMT mit Oxidator, Veresterungskolonne und Rohester-Rektifikation,

Fig. 2    eine Anlage zur Rückstandsaufbereitung der Sumpffraktion "Rückstand A" gemäß Fig. 1,

Fig. 3    eine Anlage zur Reinigung der Roh-DMT-Fraktion aus Fig. 1 zu DMT-Zwischenprodukt und Reinst-DMT,

Fig. 4    eine Anlage zur Filtrataufbereitung des "Filtrats A" gemäß Fig. 3,

Fig. 5    eine Anlage zur Hydrolyse des DMT-Zwischenproduktes zu PTA,

Fig. 6    eine Anlage zur gleichzeitigen Herstellung von PTA und PTA-p.

### Wege zur Ausfürung der Erfindung

### Beispiel 1

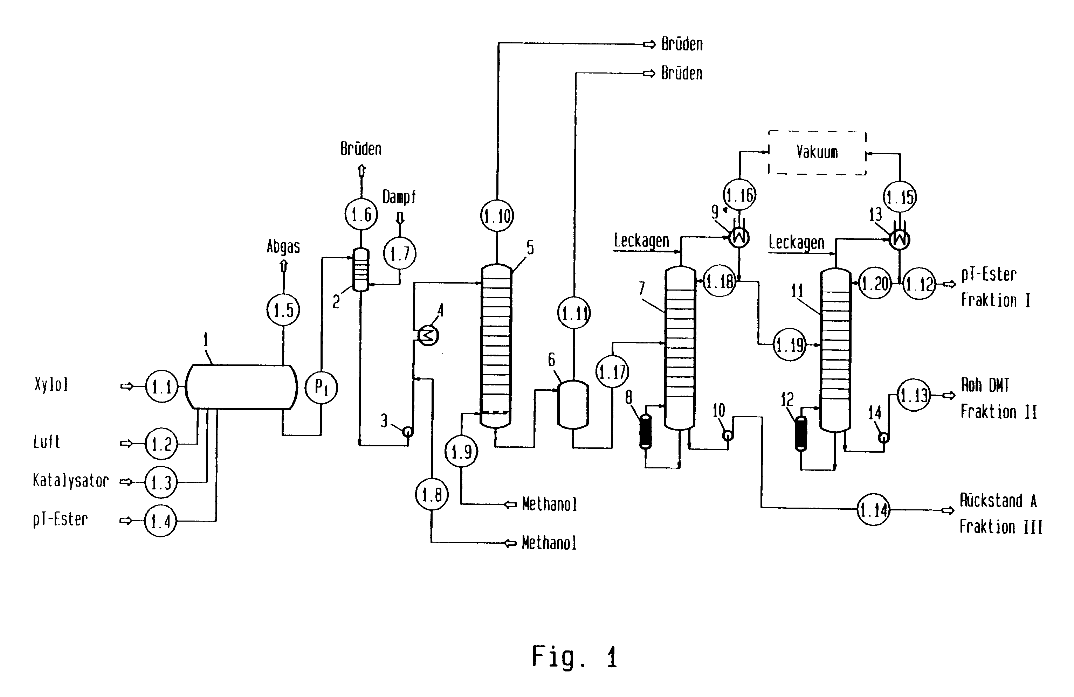

In Fig. 1 ist eine Anlage zur Herstellung von Roh-DMT (Fraktion II) nach dem erfindungsgemäßen Verfahren mit den Hauptkomponenten Oxidator 1, Veresterungskolonne 5 und Rohester-Rektifikation 7 und 11 dargestellt. Eine Analyse der nachfolgend definierten und in Fig. 1 dargestellten Stoffströme 1.1 bis 1.20 ist in Tabelle I angegeben.

Im an sich bekannten Oxidator 1, beispielsweise entsprechend der DE-C3-28 05 915, wird ein flüssiges, hauptsächlich aus p-Xylol (Stoffstrom 1.1) und p-TE (Stoffstrom 1.4) bestehendes Gemisch unter Zugabe eines Katalysators (Stoffstrom 1.3) mit Luftsauerstoff (Stoffstrom 1.2) bei einer Temperatur von ca. 160 °C und einem Druck von ca. 7 bar oxidiert.

Das entstehende Oxidat $P_1$ enthält als Hauptkomponenten p-Toluylsäure (p-TA) (ca. 17 Gew.-%), MMT (ca. 22 Gew.-%), p-Toluylsäuremethylester (p-TE) (ca. 24 Gew.-%), DMT (ca. 14 Gew.-%) und Terephthalsäure (TA) (ca. 10 Gew.-%).

Daneben findet man Nebenprodukte und Verunreinigungen, insbesondere Benzoesäuremethylester (BME), TAE, HM-BME, Schwersieder u.a.

Bei der Oxidation wird der in der Luft (Stoffstrom 1.2) enthaltene Sauerstoff bis auf Restgehalte verbraucht. Der übrig bleibende Stickstoff sättigt sich mit den in dem Oxidator 1 befindlichen Stoffen und verläßt als Oxidator-Abgas (Stoffstrom 1.5) den Oxidator 1 zusammen mit dem bei der Oxidation anfallenden Reaktionswasser sowie leichtsiedenden Crack-Produkten wie CO, $CO_2$, Ameisen- und Essigsäure.

Die als p-Xylol (Stoffstrom 1.1) bezeichnete xylolische Mischung besteht aus frisch eingesetztem Xylol mit einem Isomeren-Gehalt von 6.000 ppm sowie den aus den Stoffströmen 1.5, 1.6 und 1.10 zurückgewonnenen xylolischen bzw. organischen Fraktionen.

Der para-Toluylsäuremethylester (p-TE) (Stoffstrom 1.4) wird einem nicht dargestellten Zwischenbehälter entnommen, der vorwiegend aus dem Kopfprodukt der Rohester-Rektifikationskolonne 11 (Stoffstrom 1.12) gespeist wird. Im Ausführungsbeispiel gemäß Fig. 1 werden die organischen Anteile der Ströme 1.11, 1.16, 1.15 sowie der aus dem Rückstand 1.14 zurückgewonnenen Produkte ebenfalls in den p-TE-Zwischenbehälter eingespeist.

Im wesentlichen wird somit der p-TE-Strom 1.12 wieder in den Oxidator 1 zurückgeführt, so daß ein geschlossener Kreislauf entsteht.

Die bei der Oxidation entstehenden Reaktionsprodukte weisen z.T. hohe Schmelzpunkte auf - p-TA von ca. 180 °C, MMT von ca. 227 °C - bzw. sind praktisch nicht schmelzbar (Terephthalsäure) und nur begrenzt in den anderen Stoffen löslich, so daß die Gefahr einer Auskristallisation und damit Verstopfung besteht. Durch einen relativ hohen Anteil von im Kreislauf gefahrenem p-TE und DMT kann diese Gefahr begrenzt werden.

Der eingestellte p-TE-Überschuß sowie die im Kreislauf gefahrenen DMT-Mengen dienen zur Reaktionssteigerung und Schmelzpunkteinstellung des den Oxidator verlassenden Produktstromes $P_1$. Hierbei werden Schmelzpunkte im Bereich von 140 °C angestrebt.

Als Maß für ein ausgewogenes Verhältnis zwischen hochschmelzenden Säuren und den Schmelzpunkt und die Fließfähigkeit verbessernden anderen Produkten gilt die Säurezahl, die am Austritt des Oxidators bei 200 bis 300 g KOH/kg Oxidat gehalten wird. Bei zu hohen Säurezahlen sind schlechte Fließfähigkeit und nachteilige Schmelzpunkte zu erwarten, bei zu niedrigen Säurezahlen, d.h. zu großen Mengen an Kreislaufprodukten, zu hoher Destillationsaufwand in den Rohester-Destillationskolonnen 7 und 11. Der p-TE-Überschuß wird so eingestellt, daß die Oxidationsbedingungen im Oxidator moderat bleiben, d.h. die Temperatur zwischen 150 und 180 °C bei 5 bis 8 bar liegt. Es hat sich bewährt, im fertigen Oxidat Konzentrationen von 5 bis 30 Gew.-% p-TE einzuhalten. Die in Tabelle I dargestellten Mengenströme ergeben sich, wenn ein p-TE-Überschuß von 24 % und eine Säurezahl am Oxidatoraustritt von 225 g KOH/kg Oxidat eingehalten werden.

Das fertige Oxidat wird im Stripper 2 mit Wasserdampf (Stoffstrom 1.7) von noch enthaltenem Xylol befreit. Der Brüden, der sich ergibt, hat eine Zusammensetzung 1.6 gemäß Tabelle I.

Der Oxidatstrom $P_1$ wird, nach Druckerhöhung mit der Hochdruckpumpe 3, mit heißem Methanol (Stoffstrom 1.8) vereinigt und nach Aufheizung im Wärmetauscher 4 auf 250 °C in dem Veresterungs-Kolonnenreaktor 5 bei 250 °C mit Methanol (Stoffstrom 1.9) verestert. Das Methanol wird dampfförmig am Sumpf zugegeben. Der relativ hohe Methanolüberschuß verläßt mit dem Reaktionswasser den Kopf der Kolonne 5 (Stoffstrom 1.10) und ist gesättigt mit den am Kopf der Kolonne 5 vorhandenen anderen Verbindungen. Das veresterte Oxidat, der sogenannte Rohester, wird in dem Zwischenbehälter 6 auf Normaldruck entspannt und kühlt sich dabei auf Temperaturen um 200 °C ab. Das noch vorhandene Methanol entweicht dampfförmig (Stoffstrom 1.11) über eine Brüden-Leitung und ist gesättigt mit den im Reaktor enthaltenen Komponenten.

### Rohester – Rektifikation

Aus dem Zwischenbehälter 6 wird die Rohester-Mischung, die neben DMT (ca. 58 Gew.-%) und p-TE (ca. 33 Gew.-%) noch Nebenprodukte wie TAE, BME, Hochsieder, HM-BME sowie den Katalysator enthält, in die aus zwei Kolonnen 7, 11 bestehende Rektifikation gepumpt.

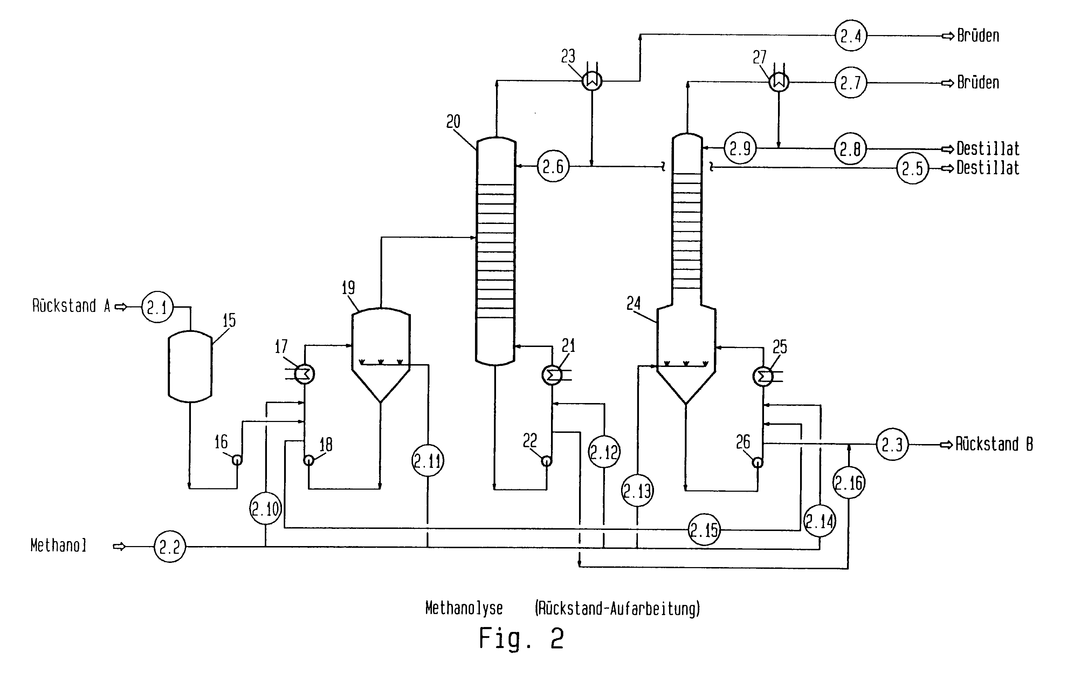

Die Schwersiederkolonne 7 mit Verdampfer 8 und Kondensator 9 dient zur Abtreibung der im Rohester enthaltenen Schwersieder als Sumpfprodukt (Stoffstrom 1.14), das als "Rückstand A" in einer separaten Rückstandsaufbereitung (Fig. 2) weiterbehandelt werden kann.

Außerdem enthält der "Rückstand A" den gesamten Katalysator sowie einen hohen Anteil von ca. 52 Gew.-% DMT. Zusammen mit der Rückstandsfraktion wird ebenfalls der größte Teil des HM-BME ausgeschleust.

Durch den vorgesehenen hohen DMT-Anteil im Sumpfstrom 1.14 der Schwersiederkolonne 7 kann die Sumpftemperatur bei 250 °C und der Kopfdruck bei 0,2 bar gehalten werden, wobei sich am Kopf der Schwersiederkolonne 7 ein hohes Temperaturniveau von ca. 205 °C einstellt, das eine energetisch günstige Rückgewinnung der Kondensationswärme (Kondensator 9) in Form von Niederdruckdampf (3,5 bar) ermöglicht. Der Kopfdruck von ca. 0,2 bar in der Schwersiederkolonne 7 wird durch eine nicht dargestellte Vakuumanlage aufrechterhalten, wobei der Stoffstrom 1.16 abgesaugt wird.

Das kondensierte Kopfprodukt fließt vom Kondensator 9 zur Abtreibung der p-TE-Fraktion in die Leichtsiederkolonne 11 mit Verdampfer 12 und Kondensator 13. Bei Temperaturen von ca. 194 °C am Kopf kann die Kondensationswärme durch Erzeugung von 3,5-bar-Niederdruckdampf abgeführt werden. Der DMT-Gehalt im Kopfprodukt wird mit ca. 18 Gew.-% so hoch gehalten, daß sich einschließlich der im Rückstand A (Stoffstrom 1.14) enthaltenen DMT-Mengen eine hohe Kreislauf-DMT-Menge und damit die angestrebte Säurezahl von 225 g KOH/kg Oxidat im Oxidat ergibt. Die im dargestellten Ausführungsbeispiel eingestellten Verhältnisse ergeben sich aus Tabelle I (Stoffstrom 1.14 und 1.12). Stellt man diese, für den Prozeß günstigen Verhältnisse durch die Wahl der Rückläufe und Mengen ein, kann man ebenfalls die in Tabelle I angegebenen Anteile von TAE und HM-BME im Roh-DMT von 0,1 Gew.-% für TAE bzw. 0,05 Gew.-% für HM-BME erreichen.

In der Leichtsiederkolonne 11 wird eine Sumpftemperatur von 250 °C, eine Kopftemperatur von 194 °C und ein Kopfdruck von 0,3 bar eingestellt. Der Kopfdruck wird durch Absaugen des Stromes 1.15 mittels einer nicht dargestellten Vakuumanlage aufrechterhalten.

Der im Kopf enthaltene p-TE-Anteil garantiert bei der vorgesehenen Produktion von DMT einen Restgehalt von p-TE im Oxidat $P_1$ von 24 %. Die für die Qualität des Roh-DMTs wichtigen Stoffe HM-BME und TAE stellen Zwischenprodukte der p-TE-Oxidation zu MMT dar und können deshalb problemlos mit dem p-TE-Destillat bzw. den aus dem Rückstand gewonnenen Wertprodukten in den Oxidator zurückgeführt werden. Aus den Rücklaufverhältnissen bzw. den Brüden-Mengen bei den Kolonnen (7,11) ist ersichtlich, daß die für die Destillation benötigten Energien relativ gering, verglichen mit dem Verfahren gemäß der DE-C2-30 11 558, liegen. Insbesondere ist vorteilhaft, daß die Kondensationswärme der Leichtsiederkolonne 11 in Form von Niederdruckdampf (3,5 bar) abgeführt werden kann.

### Umkristallisation

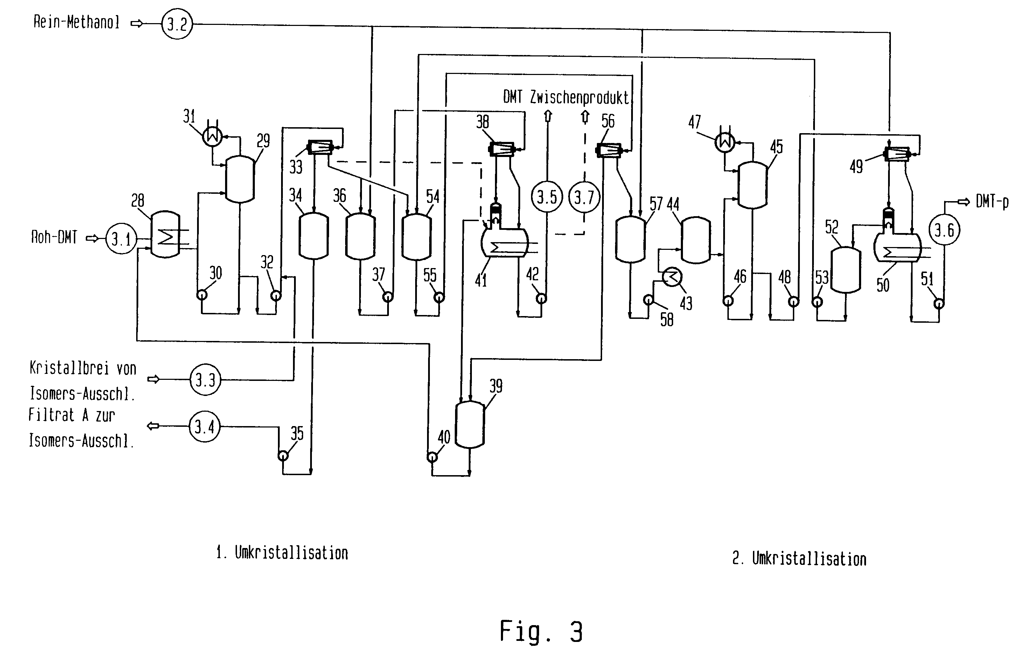

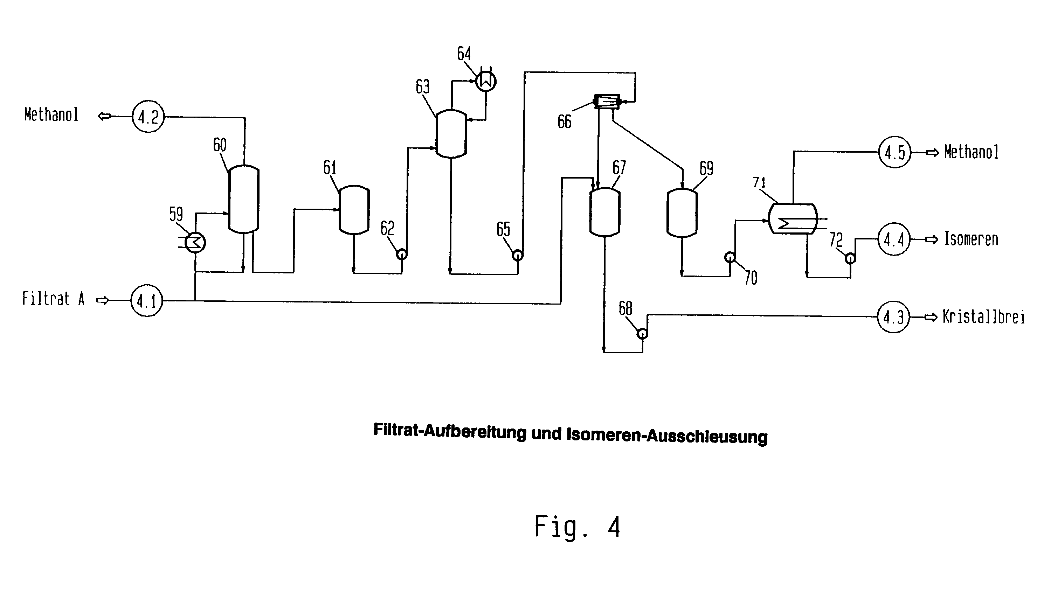

Das gemäß Fig. 1 rektifikativ gereinigte Roh-DMT (Fraktion II) wird erfindungsgemäß durch einfache methanolische Lösungsmittel-Umkristallisation zu dem DMT-Zwischenprodukt weiter gereinigt (Fig. 3, Analysen der Stoffströme 3.1 bis 3.5 siehe Tabelle III). Hierzu wird das Roh-DMT (Stoffstrom 3.1, entsprechend Stoffstrom 1.13 nach Fig. 1) im Mischbehälter 28 mit Methanol aus dem Zwischenbehälter 39 angemischt. Die heiße Lösung wird im Kristallisator 29 mit Umwälzpumpe 30 und Kondensator 31 unter Entspannung abgekühlt. Die Pumpe 32 fördert den Kristallbrei zur Zentrifuge 33, wo er in das Filtrat A und das Kristallisat zerlegt wird. Das Filtrat A wird über Behälter 34 und Pumpe 35 (Stoffstrom 3.4) zur Isomerenausschleusung gefahren (Fig. 4).

Von entscheidener Bedeutung für die Reinigungswirkung der apparativ aufwendigen Umkristallisation ist die möglichst vollständige Trennung von Umkristallisat und anhaftender Mutterlauge, da letztere mit nahezu den gesamten Verunreinigungen behaftet ist. Zur Steigerung der Reinigungswirkung der Umkristallisation wird daher im dargestellten Ausführungsbeispiel eine methanolische Wäsche des Kristallisates durchgeführt. Hierzu wird das Kristallisat im Behälter 36 mit frisch destilliertem Methanol angemischt und über eine Pumpe 37 auf die Zentrifuge 38 gegeben. Das entstehende Filtrat - ein nur leicht verunreinigtes Methanol - wird auf den Kopf des Schmelzers 41 gegeben, während das Kristallisat in den Schmelzer 41 geleitet wird.

Im Schmelzer 41 wird die Rest-Mutterlauge des Kristallisates abgetrieben und das DMT aufgeschmolzen. Das verdampfte Methanol wird im Kopf des Schmelzers 41 kondensiert und zusammen mit dem Filtrat der Zentrifuge 38 in den Methanol-Zwischenbehälter 39 geleitet.

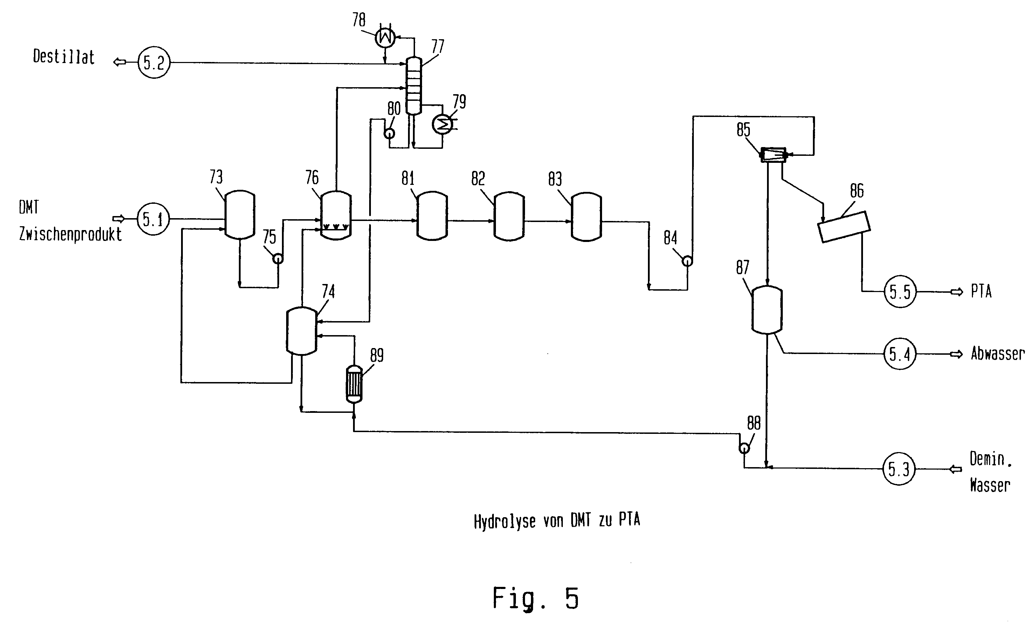

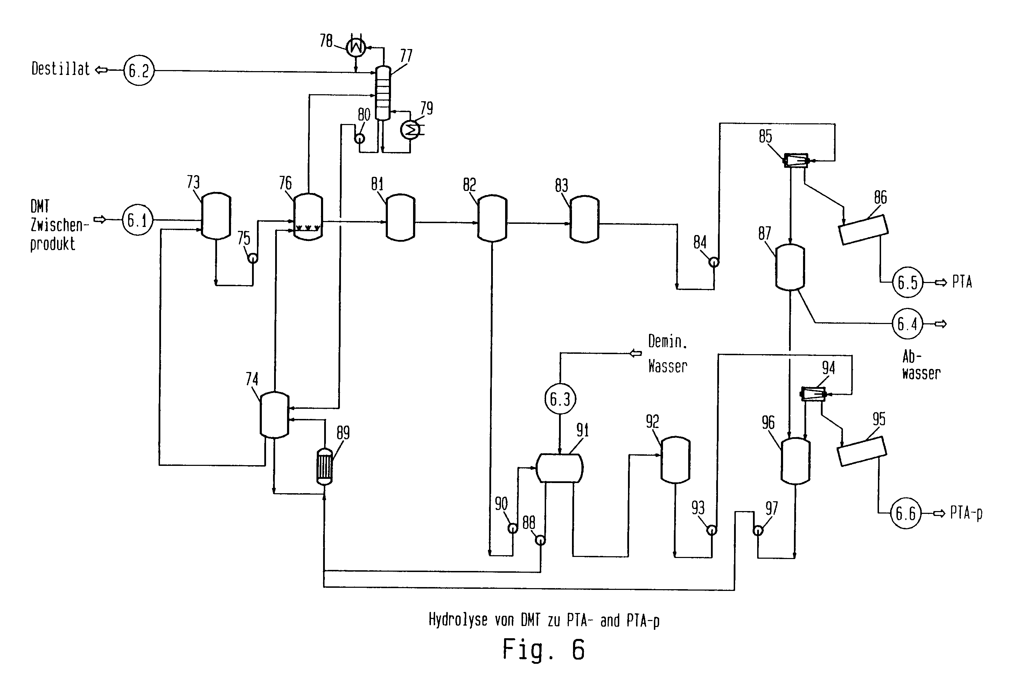

Das dem Schmelzer 41 über die Pumpe 42 entnommene DMT (Stoffstrom 3.5) stellt das DMT-Zwischenprodukt gewünschter Reinheit dar. Es kann wahlweise durch Hydrolyse (Fig. 5 und 6) zu Terephthalsäure oder durch nochmalige Umkristallisation zu faserreinem DMT (DMT-p) weiterverarbeitet werden, wobei besonders die Kombination dieser Möglichkeiten überraschende Vorteile bietet. Das DMT-Zwischenprodukt (Stoffstrom 3.5) enthält nur noch sehr geringe Verunreinigungen an TAE und HM-BME von max. 100 ppm, im dargestellten Ausführungsbeispiel von nur 17 ppm (s. Tab. III), sowie geringe Mengen der Isomeren DMI und DMO, im dargestellten Ausführungsbeispiel zusammen 67 ppm.

### Filtrataufbereitung

Das aus dem Zwischenbehälter 34 (Fig. 3) über die Pumpe 35 entnommene Filtrat (Stoffstrom 3.4, Fig. 3 = Stoffstrom 4.1, Fig. 4) wird durch Eindampfung im Verdampfer 59 mit Abscheider 60 zunächst eingedickt, so daß die eingedickte Mischung zu 2/3 aus Methanol und 1/3 aus gelöster Substanz (DMT + Verunreinigungen) besteht. Der Verdampfungsdruck liegt bei 4 bar und die Temperatur bei 100 °C. Die übrigbleibende Lösung wird unter diesem Druck im Behälter 61 zwischengelagert und mit der Pumpe 62 in den Kristallisator 63 mit Kondensator 64 gepumpt, wo die Lösung durch Entspannungskühlung auf ca. 25 °C abgekühlt wird.

Die entsprechende Suspension wird über Pumpe 65 auf die Zentrifuge 66 gegeben und in ein Filtrat und das Kristallisat aufgetrennt. Das Kristallisat kann, wie in Fig. 4 dargestellt, als im Rührbehälter 67 mit Methanol angemischte Suspension über Pumpe 68 in die erste Umkristallisation zurückgeführt werden (Stoffstrom 4.3 in Fig. 4, Stoffstrom 3.3 in Fig. 3).

Das Filtrat wird in den Sammelbehälter 69 und von dort mit der Pumpe 70 in den Verdampfer 71 geleitet, in dem das Methanol vollständig abgedampft wird (Stoffstrom 4.5, Tabelle 4). Die übrig bleibende Mischung besteht weitgehend aus DMI (37 Gew.-%) und DMO (26 Gew.-%) und enthält nur verhältnismäßig geringe Anteile an Wertprodukten, insbesondere DMT (16 Gew.-%), TAE (10 Gew.-%) sowie je 5 Gew.-% p-TA und HM-BME, so daß sie ohne große Verluste über die Pumpe 72 vollständig ausgeschleust werden (Stoffstrom 4.4), insbesondere zur Verbrennung.

### Rückstandsaufbereitung

Der "Rückstand A" gemäß Fig. 1 (Stoffstrom 1.14, Tabelle I = Stoffstrom 2.1, Tabelle II) wird im Tank 15 (Fig. 2) zwischengelagert. Der Rückstand A enthält neben DMT (ca. 52 Gew.-%) vorwiegend Schwersieder (37 Gew.-%) sowie den Katalysator (ca. 1 Gew.-%).

In der zweistufigen Methanolyse gemäß Fig. 2 werden die im Rückstand A noch vorhandenen Säuren nachverestert, ein Teil der hochsiedenden, insbesondere der zweikernigen aromatischen Verbindungen gespalten und die Wertprodukte (DMT, HM-BME, Methoxymethyl-benzoesäuremethylester [MM-BME] und p-TE) von den verbleibenden unerwünschten Schwersiedern getrennt. Die Wertprodukte werden in die Oxidation zurückgeführt, während die unerwünschten Schwersieder aus dem Prozeß ausgeschleust werden.

Unter den destillierbaren Schwersiedern im Rückstand A befinden sich Stoffe, die, sofern sie in die Oxidation zurückgeführt werden, die Ausbeute bzw. Selektivität der Oxidation nachteilig beeinflussen, da sie die Wirksamkeit des Katalysatorsystems beeinträchtigen. Des weiteren können sich während der oxidativen Behandlung solcher Stoffe Spaltprodukte, z.B. $CH_3$-DMT-Isomere, bilden, die für die Qualität des Roh-DMTs nachteilig sind, destillativ nur schwer aus dem Rohester entfernt werden können und zusätzlichen Reinigungsaufwand in der nachfolgenden Kristallisation erfordern, wenn hochreine Polyesterrohstoffe erzeugt werden sollen. Die Rückführung solcher Schwersieder in die Oxidation muß daher vermieden werden.

Im dargestellten Ausführungsbeispiel ergibt sich folgende Reaktionsführung:

Aus dem Tank 15 wird der Rückstand A von der Rohester-Destillation mit der Pumpe 16 in ein Umwälzsystem gefahren, das aus Pumpe 18, Wärmetauscher 17 und Reaktor 19 besteht. Gleichzeitig wird dampfförmiges Methanol (Stoffstrom 2.10) in den Kreislauf gepumpt. Durch die Anwesenheit des Methanols werden noch enthaltene Restmengen von nicht veresterten schwersiedenden Säuren nachverestert und vom hohen Methanoldampfüberschuß z. T. aufgenommen und dampfförmig in den Brüdenraum des Reaktors 19 geflasht. Der nicht verdampfte Flüssiganteil bleibt im Sumpfteil des Reaktors 19 zurück und wird erneut mit Methanoldampf behandelt und weiter methanolytisch gespalten. Die entstehenden Ester gelangen in die methanolische Dampfphase und werden gemeinsam mit den destillierbaren Bestandteilen des Rückstandes und dem Flashprodukt in die Destillationskolonne 20 geleitet. Die im Ausführungsbeispiel gewählte Temperatur im Reaktor 19 betrug 265 bis 270 °C, während die Temperatur in Kolonne 20 bei etwa 250 °C liegt, so daß ein Teil der schwersiedenden Bestandteile am Sumpf der Kolonne 20 abgezogen werden kann. Im Abtriebsteil der Destillationskolonne 20 werden hierzu die hochsiedenden Nebenprodukte angereichert und am Sumpf der Kolonne abgezogen. Ein Teil des hochsiedenden Sumpfproduktes wird ausgeschleust, während der Rest, mit Methanoldampf vermischt, über Pumpe 22 und Verdampfer 21 in den Sumpf der Kolonne zurückgeführt wird. Bis auf die destillierbaren Hochsieder, die keine Wertprodukte darstellen und deshalb, wie oben erwähnt, als Schwersieder den Sumpf der Kolonne 20 verlassen, werden alle Wertprodukte mit dem Methanoldampf zum Dephlegmator 23 geführt und kondensiert. Ein Teil des Kondensats wird als Rücklauf verwendet, während der Rest ausgeschleust (Stoffstrom 2.5) und dem p-TE-Strom zur Oxidation beigemengt wird. Der Methanolüberschuß (Stoffstrom 2.4) wird zusammen mit dem bei der Methanolyse und Nachveresterung anfallenden Wasser auf eine Methanol-Entwässerungskolonne (nicht dargestellt) geführt.

Ein Teil des Umwälzstromes aus Reaktor 19 wird aus dem Kreislauf ausgeschleust und im Reaktor 24 mit Wärmetauscher 25, Umwälzpumpe 26 und Dephlegmator 27 einer weiteren Methanolyse-Stufe unterworfen. Diese zweite Methanolyse-Stufe wird bei einer Temperatur von > 275 °C durchgeführt, so daß weitere hochmolekulare Verbindungen des Rückstandes A gespalten werden. Die den Reaktor 24 gasförmig verlassenden Produkte werden im Dephlegmator 27, mit Ausnahme des Methanols, vollständig kondensiert, während das Methanol (Stoffstrom 2.7) zusammen mit dem Methanol aus dem Dephlegmator 23 zur Weiterverwendung im gesamten Prozeß ausgeschleust wird. Die im Dephlegmator 27 kondensierten Produkte werden zum größten Teil zusammen mit dem p-TE-Strom in die Oxidation zurückgeführt, während ein kleiner Teil als Rücklauf in den Kopf der Kolonne des Reaktors 24 zurückgeführt wird. Am Sumpf des Reaktors 24 werden die nicht verdampften Bestandteile abgezogen und teilweise zur Katalysator-Rückgewinnung (Stoffstrom 2.3) ausgeschleust, während der Rest mit Pumpe 26 über Verdampfer 25 in den Reaktor rückgeführt wird. Zusätzlich wird in den Reaktorumlauf Methanol dampfförmig eingegeben.

### Beispiel 2

Soweit an die Reinheit des DMT-Zwischenproduktes nicht besonders hohe Anforderungen gestellt werden, z.B. zur Herstellung mittelreiner Terephthalsäure (MTA), kann auf die Wäsche des DMT-Kristallisates ggf. verzichtet werden. In Fig. 3 ist diese Alternative zur Herstellung des DMT-Zwischenproduktes gestrichelt dargestellt. Das dem Schmelzer entnommene DMT-Zwischenprodukt (Stoffstrom 3.7) enthält noch ca. 150 ppm an TAE und HM-BME sowie 580 ppm an Isomeren DMI und DMO.

### Beispiel 3

In Fig. 3 ist weiterhin die gleichzeitige Herstellung von faserreinem DMT-p und DMT-Zwischenprodukt zur Terephthalsäure-Produktion dargestellt. Hierzu wird der Kristallisatstrom der Zentrifuge 33 auf die Anmischbehälter 36 und 54 aufgeteilt. Im Anmischbehälter 54 wird das DMT-Kristallisat mit Methanol aus dem Zwischenbehälter 52 angemischt und über die Pumpe 55 auf die Zentrifuge 56 gegeben. Das entstehende Filtrat wird dem Methanol-Zwischenbehälter 39 zugeführt, während das Kristallisat im Anmischbehälter 57 mit Rein-Methanol angemischt und über die Pumpe 58 und Wärmetauscher 43 in den Zwischenbehälter 44 gegeben wird. In der nachfolgenden, aus Kristallisator 45, Wärmetauscher 47 und Pumpe 46 bestehenden zweiten Umkristallisation wird das in Methanol gelöste DMT nochmals gereinigt. Die Kristallsuspension wird anschließend über die Pumpe 48 auf die Wasch-Zentrifuge 49 gegeben, wo sie in einen Filtrat- und einen Kristallisatstrom aufgeteilt wird. Das Kristallisat wird anschließend im Schmelzer 50 aufgeschmolzen und von Rest-Mutterlauge befreit, während das Filtrat über den Kondensationskopf des Schmelzers 50 in den Zwischenbehälter 52 geführt wird. Das nahezu ausschließlich aus Methanol bestehende Filtrat wird über die Pumpe 53 in den Anmischbehälter 54 zurückgeführt (Gegenstrom). Das erzeugte DMT-p wird mittels der Pumpe 51 dem Schmelzer 50 entnommen. Es enthält weniger als 10 ppm an Isomeren und nur etwa 2 ppm TAE und HM-BME (Stoffstrom 3.6) und stellt damit ein faserreines DMT mit - für großtechnische Verfahren - bislang unerreichter Reinheit dar.

**Beispiel 4**

*Hydrolyse (PTA)*

Die Hydrolyse des DMT-Zwischenproduktes gemäß Fig. 3 (Stoffstrom 3.5) zu faserreiner Terephthalsäure (PTA) wird anhand Fig. 5 näher erläutert.

Im Mischbehälter 73 wird das von der ersten Umkristallisation (Fig. 3) kommende DMT-Zwischenprodukt (Stoffstrom 5.1) mit Wasser aus einem Strippdampferzeuger 74 im Verhältnis 1 : 1 gemischt und teilweise hydrolisiert. Mit der Pumpe 75 wird die Suspension in den Hydrolyse-Reaktor 76 gepumpt, in dem das Gemisch mit Strippdampf aus dem Strippdampferzeuger 74 behandelt wird. Hierdurch wird das bei der Hydrolyse frei werdende Methanol zusammen mit Wasserdampf und dem gebildeten Dimethylether in die Hydrolysekolonne 77 mit Kondensator 78 und Wärmetauscher 79 abgetrieben. Durch geeignete Wahl des Rücklaufverhältnisses wird erreicht, daß am Sumpf der Kolonne 77 mit Hilfe der Pumpe 80 ein methanolfreies Wasser abgezogen und in den Strippdampferzeuger zurückgeleitet werden kann. Das kondensierte Kopfprodukt (Stoffstrom 5.2) besteht hauptsächlich aus Dimethylether (DME), Methanol und Wasser und kann, nach destillativer Abtrennung des DME, einer (nicht dargestellten) Methanol-Rektifikation zugeführt werden. Der Dimethylether wird verbrannt, während das Methanol in die Veresterung (Fig. 1) zurückgeführt wird. Vom Reaktor 76 wird in einer dreistufigen Entspannungskristallisation (Kristallisatoren 81, 82 und 83) die entstandene Terephthalsäure auskristallisiert und mit der Pumpe 84 auf die Zentrifuge 85 gefördert. Der feuchte Kristallbrei wird dem Trockner 86 zugeführt und dort zu faserreiner Terephthalsäure (PTA) getrocknet (Stoffstrom 5.5). Die von der Zentrifuge 85 in den Sammelbehälter 87 ablaufende Mutterlauge wird zur Entfernung von noch gelösten Isomeren (ITA, OTA) und von Fremdstoffen, die z.B. durch Abrieb aus den mechanisch bewegten Teilen wie Gleitringdichtungen etc. entstehen, teilweise als Abwasser ausgeschleust (Stoffstrom 5.4). Der größte Teil des Filtrats wird jedoch mit der Pumpe 88 in den Strippdampferzeuger mit Wärmetauscher 89 zurückgeführt. Das in der Hydrolyse verbrauchte und im Destillat (Stoffstrom 5.2) sowie im Abwasser (Stoffstrom 5.4) abgezogene Wasser wird durch frisches demineralisiertes Wasser (Stoffstrom 5.3) ersetzt.

Die Temperatur im Mischbehälter 73 und im Hydrolyse-Reaktor 76 betrug im dargestellten Ausführungsbeispiel ca. 265 °C. Die Entspannungs-Kristallisation wurde bis zu einer Temperatur von 95 °C geführt. Wie der Tabelle V zu entnehmen ist (Stoffstrom 5.5), weist die PTA neben ca. 100 ppm MMT nur noch ca. 40 ppm an weiteren Verunreinigungen auf und ist damit für fast alle Anwendungszwecke geeignet.

**Beispiel 5**

*Hydrolyse (PTA−p)*

Falls eine Terephthalsäure mit bislang unerreicht geringem MMT-Gehalt (PTA-p) gewünscht wird, kann die PTA im Anschluß an die Auskristallisation ggf. nochmals mit demineralisiertem Wasser gewaschen werden. Besonders vorteilhaft ist die kombinierte Herstellung von PTA und PTA-p gemäß Fig. 6. Die Vorrichtungen 73 bis 89 und die Verfahrensweise entsprechen dabei weitgehend Fig. 5. Lediglich im Kristallisator 82 wird ein Teil des Kristallbreis mit Pumpe 90 abgezogen und in den Gegenstromwäscher 91, z. B. gemäß der DE-A1-36 39 958, gepumpt, wo er im Gegenstrom mit demineralisiertem Wasser (Stoffstrom 6.3) gewaschen wird. Die Wäsche findet bei einer Temperatur von 190 °C statt. Die gewaschene Kristallsuspension wird in dem Kristallisator 92 entspannt und von dort mit Pumpe 93 auf die Zentrifuge 94 zur Abtrennung der Mutterlauge gefördert. Die feuchte PTA wir im Trockner 95 getrocknet und verläßt als hochreine PTA-p mit einem Gesamtgehalt an Verunreinigungen einschließlich MMT von < 50 ppm (Stoffstrom 6.6, Tab. 6) die Anlage, während die von der Zentrifuge 94 ablaufende Mutterlauge im Behälter 96 mit der von der Zentrifuge 85 ablaufenden Mutterlauge vereinigt und mit der Pumpe 97 in den Strippdampferzeuger 74 gepumpt wird.

Pumpe 88 fördert die Mutterlauge aus der Gegenstromwäsche 91 in den Strippdampferzeuger 74 zurück.

## Abkürzungen

| | |
|---|---|
| DME - | Dimethylether |
| DMI - | Dimethylisophthalat |
| DMO - | Dimethylorthophthalat |
| DMT - | Dimethylterephthalat |
| DMT-p - | Reinst-DMT |
| HM-BME - | Hydroxymethyl-benzoesäuremethylester |
| ITA - | Isophthalsäure |
| MM-BME - | Methoxymethyl-benzoesäuremethylester |
| MMT - | Monomethylterephthalat |
| MTA - | mittelreine Terephthalsäure |
| OTA - | Orthophthalsäure |
| p-TA - | para-Toluylsäure |
| p-TE - | para-Toluylsäure-methylester (pT-Ester) |
| p-X - | para-Xylol |
| PTA - | Terephthalsäure hoher Reinheit |
| PTA-p - | Terephthalsäure höchster Reinheit (Gehalt an MMT und p-TA von zusammen < 50 ppm) |
| TA - | Terephthalsäure |
| TAE - | Terephthalaldehydsäure-methylester |
| TAS - | Terephthalaldehydsäure (4-CBA) |

## Definitionen

In der Beschreibung verwendete %- und ppm-Angaben beziehen sich, soweit nicht anders angegeben, auf Gewichtsanteile.

## Legende

| | |
|---|---|
| 1 - | Oxidator |
| 2 - | Stripper |
| 3 - | Hochdruckpumpe |
| 4 - | Wärmetauscher |
| 5 - | Veresterungskolonne |
| 6 - | Zwischenbehälter |
| 7 - | Rohester-Rektifikation (Schwersiederkolonne) |
| 8 - | Verdampfer |
| 9 - | Kondensator |
| 10- | Pumpe |
| 11- | Rohester-Rektifikation (Leichtsiederkolonne) |
| 12- | Verdampfer |
| 13- | Kondensator |
| 14- | Pumpe |
| 15- | Tank |
| 16- | Pumpe |
| 17- | Wärmetauscher |
| 18- | Pumpe |
| 19- | Reaktor |
| 20- | Destillationskolonne |
| 21- | Verdampfer |
| 22- | Pumpe |
| 23- | Dephlegmator |

| | |
|---|---|
| 24- | Reaktor |
| 25- | Wärmetauscher |
| 26- | Umwälzpumpe |
| 27- | Dephlegmator |
| 28- | Mischbehälter |
| 29- | Kristallisator |
| 30- | Umwälzpumpe |
| 31- | Kondensator |
| 32- | Pumpe |
| 33- | Zentrifuge |
| 34- | Behälter |
| 35 - | Pumpe |
| 36 - | Behälter |
| 37 - | Pumpe |
| 38 - | Zentrifuge |
| 39 - | Methanol-Zwischenbehälter |
| 40 - | Pumpe |
| 41 - | Schmelzer |
| 42 - | Pumpe |
| 43 - | Wärmetauscher |
| 44 - | Zwischenbehälter |
| 45 - | Kristallisator |
| 46 - | Pumpe |
| 47 - | Wärmetauscher |
| 48 - | Pumpe |
| 49 - | Waschzentrifuge |
| 50 - | Schmelzer |
| 51 - | Pumpe |
| 52 - | Zwischenbehälter |
| 53 - | Pumpe |
| 54 - | Anmischbehälter |
| 55 - | Pumpe |
| 56 - | Zentrifuge |
| 57 - | Anmischbehälter |
| 58 - | Pumpe |
| 59 - | Verdampfer |
| 60 - | Abscheider |
| 61 - | Behälter |
| 62 - | Pumpe |
| 63 - | Kristallisator |
| 64 - | Kondensator |
| 65 - | Pumpe |
| 66 - | Zentrifuge |
| 67 - | Rührbehälter |
| 68 - | Pumpe |
| 69 - | Sammelbehälter |
| 70 - | Pumpe |
| 71 - | Verdampfer |
| 72 - | Pumpe |
| 73 - | Mischbehälter |
| 74 - | Strippdampferzeuger |
| 75 - | Pumpe |
| 76 - | Hydrolyse-Reaktor |
| 77 - | Hydrolyse-Kolonne |
| 78 - | Kondensator |
| 79 - | Wärmetauscher |
| 80 - | Pumpe |
| 81 - | Kristallisator |

11

82 -      Kristallisator
83 -      Kristallisator
84 -      Pumpe
85 -      Zentrifuge
86 -      Trockner
87 -      Sammelbehälter
88 -      Pumpe
89 -      Wärmetauscher
90 -      Pumpe
91 -      Gegenstromwäscher
92 -      Kristallisator
93 -      Pumpe
94 -      Zentrifuge
95 -      Trockner
96 -      Behälter
97 -      Pumpe

EP 0 464 046 B1

Tabelle I

| Analysen-Nr. / Komponenten | 1.1 Xylol von der Separation z. Oxi. kg/h | Ma % | 1.2 Prozessluft zur Oxidation kg/h | Ma % | 1.3 Katalysator zur Oxidation kg/h | Ma % | 1.4 pT-Ester zur Oxidation kg/h | Ma % | 1.5 Abgas der Oxidation kg/h | Ma % |
|---|---|---|---|---|---|---|---|---|---|---|
| Schwersieder | - | - | | | | | - | - | - | - |
| DMT+DMI+DMO | 191,8 | 0,58 | | | | | 11244,6 | 22,48 | 118,7 | 0,12 |
| MM-BME | | | | | | | 379,2 | 0,76 | | |
| MMT | | | | | | | 516,1 | 1,03 | | |
| HM-BME | | | | | | | 499,1 | 0,99 | | |
| TA | | | | | | | - | - | | |
| TAE | 15,8 | 0,05 | | | | | 1144,2 | 2,29 | 15,8 | 0,02 |
| p-TE | 5189,0 | 15,63 | | | | | 32473,3 | 64,92 | 2137,1 | 2,21 |
| TAS | - | - | | | | | - | - | | |
| p-TA | 102,9 | 0,31 | | | | | - | - | 102,9 | 0,11 |
| BME | 2239,5 | 6,75 | | | | | 3556,6 | 7,11 | 1266,4 | 1,31 |
| Benzoesäure | - | - | | | | | | | - | - |
| p-X | 25318,5 | 76,28 | | | | | | | 5960,1 | 6,17 |
| Essigsäure | | | | | | | | | 441,8 | 0,46 |
| Kobalt | | | | | 32,08 | 6,91 | | | - | - |
| Mangan | | | | | 7,76 | 1,68 | | | - | - |
| Ameisensäure | | | | | | | | | 220,9 | 0,23 |
| Kohlendioxid | | | | | | | | | 1850,8 | 1,92 |
| Methanol | | | | | | | 136,9 | 0,27 | 474,9 | 0,49 |
| Sauerstoff | | | 21720,4 | 22,86 | | | | | 2732,3 | 2,83 |
| Stickstoff | | | 71902,1 | 75,69 | | | | | 71902,1 | 74,51 |
| Kohlenmonoxid | | | - | - | | | | | 462,7 | 0,48 |
| Wasser | 133,5 | 0,40 | 1377,5 | 1,45 | 424,16 | 91,41 | 69,0 | 0,15 | 7915,2 | 8,20 |
| Leichtsieder | - | - | - | - | - | - | - | - | 900,3 | 0,94 |
| Σ Summe | 33191 | 100,00 | 95000 | 100,00 | 464 | 100,00 | 50019 | 100,00 | 96502 | 100,00 |

13

## Tabelle I

| Analysen-Nr. Komponenten | 1.6 Brüden von Stripper | | 1.7 Dampf zum Stripper | | P₁ Oxidat vor Stripper | | | | | |
|---|---|---|---|---|---|---|---|---|---|---|
| | kg/h | Ma % | kg/h | Ma % | kg/h | Ma % | kg/h | Ma % | kg/h | Ma % |
| Schwersieder | | | | | 2062,5 | 2,51 | | | | |
| DMT+DMI+DMO | | | | | 11101,4 | 13,51 | | | | |
| MM-BME | | | | | - | - | | | | |
| MMT | | | | | 18466,4 | 22,47 | | | | |
| HM-BME | | | | | 164,3 | 0,20 | | | | |
| TA | | | | | 8307,6 | 10,11 | | | | |
| TAE | | | | | 394,4 | 0,48 | | | | |
| p-TE | 616,2 | 9,34 | | | 19722,3 | 24,00 | | | | |
| TAS | | | | | 714,9 | 0,87 | | | | |
| p-TA | 308,1 | 4,67 | | | 14330,8 | 17,44 | | | | |
| BME | | | | | 4839,9 | 5,89 | | | | |
| Benzoesäure | | | | | 82,2 | 0,10 | | | | |
| p-X | 1232,4 | 18,68 | | | 1232,6 | 1,5 | | | | |
| Essigsäure | 164,3 | 2,49 | | | 164,3 | 0,20 | | | | |
| Kobalt | - | - | | | 10,7 | 0,013 | | | | |
| Mangan | - | - | | | 2,5 | 0,003 | | | | |
| Ameisensäure | 82,2 | 1,25 | | | 82,2 | 0,10 | | | | |
| Kohlendioxid | | | | | | | | | | |
| Methanol | | | | | | | | | | |
| Sauerstoff | | | | | | | | | | |
| Stickstoff | | | | | | | | | | |
| Kohlenmonoxid | | | | | | | | | | |
| Wasser | 4193,8 | 63,57 | 3921 | 100,00 | 493,0 | 0,6 | | | | |
| Leichtsieder | - | - | | | - | - | | | | |
| Σ Summe | 6597 | 100,00 | 3921 | 100,00 | 82172 | 100,00 | | | | |

EP 0 464 046 B1

Tabelle I

| Analysen-Nr. | 1.8 Methanol zum Oxidataufheizer | | 1.9 Methanolbrüden zur Veresterung | | 1.10 Brüden der Veresterung | | 1.11 Brüden der Rohesterentspannung | | | |
|---|---|---|---|---|---|---|---|---|---|---|
| Komponenten | kg/h | Ma % | kg/h | Ma % | kg/h | Ma % | kg/h | Ma % | kg/h | Ma % |
| Schwersieder | | | | | – | – | – | – | | |
| DMT+DMI+DMO | | | | | 73,1 | 0,26 | 1602,3 | 7,50 | | |
| MM-BME | | | | | | | – | – | | |
| MMT | | | | | | | 516,1 | 2,42 | | |
| HM-BME | | | | | | | 98,0 | 0,46 | | |
| TA | | | | | | | – | – | | |
| TAE | | | | | | | 47,6 | 0,22 | | |
| p-TE | | | | | 2435,7 | 8,81 | 10853,7 | 50,83 | | |
| TAS | | | | | – | – | – | – | | |
| p-TA | | | | | – | – | – | – | | |
| BME | | | | | 665,0 | 2,41 | 1539,4 | 7,21 | | |
| Benzoesäure | | | | | | | | | | |
| p-X | | | | | | | | | | |
| Essigsäure | | | | | | | | | | |
| Kobalt | | | | | | | | | | |
| Mangan | | | | | | | | | | |
| Ameisensäure | | | | | | | | | | |
| Kohlendioxid | | | | | | | | | | |
| Methanol | 3613,0 | 100,00 | 32154,0 | 100,00 | 18562,9 | 67,15 | 6501,4 | 30,45 | | |
| Sauerstoff | | | | | | | | | | |
| Stickstoff | | | | | | | | | | |
| Kohlenmonoxid | | | | | | | | | | |
| Wasser | | | | | 5825,6 | 21,07 | 194,5 | 0,91 | | |
| Leichtsieder | | | | | 82,7 | 0,30 | – | – | | |
| | | | | | | | | | | |
| | | | | | | | | | | |
| | | | | | | | | | | |
| Σ Summe | 3613,0 | 100,00 | 32154,0 | 100,00 | 27645 | 100,00 | 21353 | 100,00 | | |

# Tabelle I

| Analysen-Nr. Komponenten | 1.12 pT-Ester Destillat der DMT-Roh Kolonne | | 1.13 DMT-Roh zur Umkristallisation | | 1.14 Rückstand A | | 1.15 Vakuum der DMT-Roh Kolonne | | 1.16 Vakuum der Roh-Ester Kolonne | |
|---|---|---|---|---|---|---|---|---|---|---|
| | kg/h | Ma % | kg/h | Ma % | kg/h | Ma % | kg/h | Ma % | kg/h | Ma % |
| Schwersieder | - | - | - | - | 2261,6 | 36,83 | - | - | - | - |
| DMT+DMI+DMO | 4789,8 | 18,16 | 29641,7 | 99,80 | 3167,0 | 51,57 | 47,5 | 2,19 | 265,9 | 13,13 |
| MM-BME | - | - | - | - | - | - | | | | |
| MMT | - | - | - | - | 610,7 | 9,94 | | | | |
| HM-BME | - | - | 14,8 | 0,05 | 51,5 | 0,84 | | | | |
| TA | - | - | 29,7 | 0,10 | | | | | | |
| TAE | 1053,2 | 4,00 | | | | | 26,5 | 1,22 | 16,9 | 0,83 |
| p-TE | 18788,8 | 71,34 | - | - | | | 1643,6 | 75,59 | 1107,7 | 54,71 |
| TAS | - | - | - | - | | | - | - | - | - |
| p-TA | - | - | 14,8 | 0,05 | | | | | | |
| BME | 1704,2 | 6,47 | | | | | 352,4 | 16,21 | 262,0 | 12,94 |
| Benzoesäure | | | | | | | | | | |
| p-X | | | | | | | | | | |
| Essigsäure | | | | | | | | | | |
| Kobalt | | | | | 40,4 | 0,66 | | | | |
| Mangan | | | | | 9,8 | 0,16 | | | | |
| Ameisensäure | | | | | | | | | | |
| Kohlendioxid | | | | | | | | | | |
| Methanol | | | | | | | 41,1 | 1,89 | 290,2 | 14,33 |
| Sauerstoff | | | | | | | - | - | - | - |
| Stickstoff | | | | | | | 56,0 | 2,58 | 56,0 | 2,77 |
| Kohlenmonoxid | | | | | | | - | - | - | - |
| Wasser | | | | | | | 6,9 | 0,32 | 26,3 | 1,29 |
| Leichtsieder | | | | | | | - | - | - | - |
| Σ Summe | 26336,0 | 100,00 | 29701,0 | 100,00 | 6141,0 | 100,00 | 2174,0 | 100,00 | 2025,0 | 100,00 |

# Tabelle I

| Analysen-Nr. Komponenten | 1.17 Feed der Roh-Ester Kolonne | | 1.18 Rücklauf der Roh-Ester Kolonne | | 1.19 Destillat der Roh-Ester Kolonne | | 1.20 Rücklauf der DMT-Roh Kolonne | |
|---|---|---|---|---|---|---|---|---|
| | kg/h | Ma % | kg/h | Ma % | kg/h | Ma % | kg/h | Ma % |
| Schwersieder | 2261,6 | 3,41 | - | - | - | - | - | - |
| DMI+DMI+DMO | 37911,9 | 57,21 | 17079,1 | 59,29 | 34479,0 | 59,29 | 7350,03 | 18,19 |
| MH-BME | - | - | - | - | - | - | - | - |
| MMT | 610,7 | 0,92 | - | - | - | - | - | - |
| HM-BME | 66,3 | 0,10 | 7,3 | 0,03 | 14,8 | 0,03 | - | - |
| TA | - | - | - | - | - | - | - | - |
| TAE | 1126,3 | 1,70 | 549,5 | 1,91 | 1109,4 | 1,91 | 1616,28 | 4,00 |
| p-TE | 21540,1 | 32,51 | 10121,2 | 35,13 | 20432,4 | 35,13 | 28826,35 | 71,34 |
| TAS | - | - | - | - | - | - | - | - |
| p-TA | 14,8 | 0,02 | 7,3 | 0,03 | 14,8 | 0,03 | - | - |
| BME | 2318,6 | 3,50 | 1018,8 | 3,53 | 2056,6 | 3,53 | 2614,34 | 6,47 |
| Benzoesäure | - | - | | | | | | |
| p-X | - | - | | | | | | |
| Essigsäure | - | - | | | | | | |
| Kobalt | 40,4 | 0,06 | | | | | | |
| Mangan | 9,8 | 0,02 | | | | | | |
| Ameisensäure | - | - | | | | | | |
| Kohlendioxid | - | - | | | | | | |
| Methanol | 331,5 | 0,50 | 20,4 | 0,07 | 9,841,4 | 0,07 | | |
| Sauerstoff | - | - | - | - | - | - | | |
| Stickstoff | - | - | - | - | - | - | | |
| Kohlenmonoxid | - | - | - | - | - | - | | |
| Wasser | 33,2 | 0,05 | 3,4 | 0,01 | 6,9 | 0,01 | | |
| Leichtsieder | - | - | | | - | - | | |
| Σ Summe | 66265,0 | 100,00 | 28807,0 | 100,00 | 58155,0 | 100,00 | 40407,0 | 100,00 |

# Tabelle II

| Komponenten | 2.1 Rückstand A von Roh-Ester Kolonne | | 2.2 Methanol von Filtrat-Eindampfung | | 2.3 Rückstand B zur Kat.-Rückgew. | | 2.4 Brüden der Nach-veresterungs-Kolonne | | 2.5 Destillat der Nach-veresterungs-Kolonne | |
|---|---|---|---|---|---|---|---|---|---|---|
| Analysen-Nr. | kg/h | Ma % | kg/h | Ma % | kg/h | Ma % | kg/h | Ma % | kg/h | Ma % |
| Schwersieder | 2261,6 | 36,83 | | | 876,1 | 91,73 | | | - | - |
| DMT+DMI+DMO | 3167,0 | 51,57 | | | 28,7 | 3,01 | 436,6 | 6,04 | 3063,0 | 96,93 |
| MM-BME | - | - | | | | | - | - | - | - |
| MMT | 610,7 | 9,94 | | | | | - | - | - | - |
| HM-BME | 51,5 | 0,84 | | | | | 2,7 | 0,04 | 44,5 | 1,41 |
| TA | | | | | | | - | - | - | - |
| TAE | | | | | | | - | - | - | - |
| p-TE | | | | | | | 1,7 | 0,02 | 1,7 | 0,05 |
| TAS | | | | | | | | | | |
| p-TA | | | | | | | | | | |
| BME | | | | | | | | | | |
| Benzoesäure | | | | | | | | | | |
| p-X | | | | | | | | | | |
| Essigsäure | | | | | | | | | | |
| Kobalt | 40,4 | 0,66 | | | 40,4 | 4,23 | | | | |
| Mangan | 9,8 | 0,16 | | | 9,8 | 1,03 | | | | |
| Ameisensäure | | | | | | | | | | |
| Kohlendioxid | | | | | | | | | | |
| Methanol | | | 13238,0 | 100,00 | | | 6766,6 | 93,55 | 50,3 | 1,59 |
| Sauerstoff | | | | | | | - | - | - | - |
| Stickstoff | | | | | | | - | - | - | - |
| Kohlenmonoxid | | | | | | | - | - | - | - |
| Wasser | | | | | | | 25,4 | 0,35 | 0,5 | 0,02 |
| Leichtsieder | | | | | | | - | - | - | - |
| Σ Summe | 6141,0 | 100,00 | 13238,0 | 100,00 | 955,0 | 100,00 | 7233,0 | 100,00 | 3160,0 | 100,00 |

18

# Tabelle II

| Analysen-Nr. Komponenten | 2.6 Rückfluss der Nach-Veresterungs-Kolonne | | 2.7 Brüden der Brüden-Destillations-Kolonne | | 2.8 Destillat der Brüden-Destillations-Kolonne | | 2.9 Rückfluss der Brüden-Destillations-Kolonne | | 2.15 Rückstand vom Nach-veresterungs-Reaktor | |
|---|---|---|---|---|---|---|---|---|---|---|
| | kg/h | Ma % | kg/h | Ma % | kg/h | Ma % | kg/h | Ma % | kg/h | Ma % |
| Schwersieder | - | - | | | | | | | 1966,9 | 78,58 |
| DMT+DMI+DMO | 2322,5 | 96,93 | 204,3 | 3,15 | 834,6 | 53,95 | 1733,4 | 53,45 | 474,6 | 18,96 |
| MM-BME | - | - | 34 | 0,52 | 345,1 | 22,31 | 716,8 | 22,31 | - | - |
| MMT | - | | - | | - | | - | | - | - |
| HM-BME | 33,7 | 1,41 | 38,6 | 0,60 | 313,6 | 20,27 | 651,3 | 20,27 | 7,4 | 0,30 |
| TA | - | - | - | - | - | - | - | - | | |
| TAE | - | - | - | - | - | - | - | - | | |
| p-TE | 1,3 | 0,05 | 50,1 | 0,77 | 26,2 | 1,69 | 54,3 | 1,69 | | |
| TAS | | | | | | | | | | |
| p-TA | | | | | | | | | | |
| BME | | | | | | | | | | |
| Benzoesäure | | | | | | | | | | |
| p-X | | | | | | | | | | |
| Essigsäure | | | | | | | | | | |
| Kobalt | | | | | | | | | 40,4 | 1,61 |
| Mangan | | | | | | | | | 9,8 | 0,39 |
| Ameisensäure | | | | | | | | | - | - |
| Kohlendioxid | | | | | | | | | - | - |
| Methanol | 38,1 | 1,59 | 6142,0 | 94,73 | 27,5 | 1,78 | 57,2 | 1,78 | 3,9 | 0,16 |
| Sauerstoff | - | - | - | - | | | | | | |
| Stickstoff | - | - | - | - | | | | | | |
| Kohlenmonoxid | - | - | - | - | | | | | | |
| Wasser | 0,4 | 0,02 | 15,0 | 0,23 | | | | | | |
| Leichtsieder | - | - | - | - | | | | | | |
| Σ Summe | 2396,0 | 100,00 | 6484,0 | 100,00 | 1547,0 | 100,00 | 3213,0 | 100,00 | 2503,0 | 100,00 |

Tabelle II

| Analysen-Nr. Komponenten | 2.16 Rückstand der Veresterungs-Kolonne kg/h | Ma % | kg/h | Ma % |
|---|---|---|---|---|
| Schwersieder | 142,5 | 95,00 | | |
| DMT+DMI+DMO | 7,5 | 5,00 | | |
| MM-BME | | | | |
| MMT | | | | |
| HM-BME | | | | |
| TA | | | | |
| TAE | | | | |
| p-TE | | | | |
| TAS | | | | |
| p-TA | | | | |
| BME | | | | |
| Benzoesäure | | | | |
| p-X | | | | |
| Essigsäure | | | | |
| Kobalt | | | | |
| Mangan | | | | |
| Ameisensäure | | | | |
| Kohlendioxid | | | | |
| Methanol | | | | |
| Sauerstoff | | | | |
| Stickstoff | | | | |
| Kohlenmonoxid | | | | |
| Wasser | | | | |
| Leichtsieder | | | | |
| Σ Summe | 150,0 | 100,00 | | |

| 2.10 kg/h | 2.11 kg/h | 2.12 kg/h | 2.13 kg/h | 2.14 kg/h |
|---|---|---|---|---|
| 1530 | 4580 | 795 | 4743 | 1590 |

Methanol-Brüden
siehe Analyse 2.2

# Tabelle III

| Analysen-Nr. / Komponenten | 3.1 DMT-Roh zur 1.Umkristallisation | | 3.2 Rein-Methanol zur Umkristallisation | | 3.3 Kristallbrei von Isomeren-Ausschl. | | 3.4 Filtrat A zur Isomeren-Ausschl. | | | |
|---|---|---|---|---|---|---|---|---|---|---|
| | kg/h | Ma % | kg/h | Ma % | kg/h | Ma % | kg/h | Ma % | kg/h | Ma % |
| DMT | 29463.4 | 99.2 | | | 1172.9 | 35.16 | 1219.4 | 2.05 | | |
| DMI | 104.0 | 0.35 | | | 21.95 | 0.66 | 125.07 | 0.21 | | |
| DMO | 74.3 | 0.25 | | | 15.55 | 0.47 | 89.22 | 0.15 | | |
| HM-BME | 14.8 | 0.05 | | | 3.15 | 0.09 | 17.78 | 0.03 | | |
| TAE | 29.7 | 0.10 | | | 6.30 | 0.19 | 35.75 | 0.06 | | |
| p-TA | 14.8 | 0.05 | | | 3.15 | 0.09 | 17.78 | 0.03 | | |
| Methanol | - | - | 55873 | 100.00 | 2113.0 | 63.34 | 57986.0 | 97.47 | | |
| Σ Summe | 29701.0 | 100.00 | 55873 | 100.00 | 3336.0 | 100.00 | 59491.0 | 100.00 | | |

21

# Tabelle III

| Analysen-Nr. Komponenten | 3.5 Zwischen-Produkt zur Hydrolyse | | 3.6 DMT-p aus Schmelzer | | 3.7 Zwischen-Produkt zur Hydrolyse | | | | | |
|---|---|---|---|---|---|---|---|---|---|---|
| | kg/h | Ma % | kg/h | Ma % | kg/h | Ma % | kg/h | Ma % | kg/h | Ma % |
| DMT | 21917,0 | 99,991 | 7499,9 | 99,999 | 21902,14 | 99,92 | | | | |
| DMI | 0,85 | 39 ppm | 0,03 | 4,3 ppm | 7,38 | 337 ppm | | | | |
| DMO | 0,61 | 28 ppm | 0,02 | 3,1 ppm | 5,27 | 240 ppm | | | | |
| HM-BME | 0,15 | 7 ppm | Spuren | 0,7 ppm | 1,05 | 48 ppm | | | | |
| TAE | 0,24 | 11 ppm | Spuren | 1,2 ppm | 2,11 | 96 ppm | | | | |
| p-TA | 0,15 | 7 ppm | Spuren | 0,7 ppm | 1,05 | 48 ppm | | | | |
| Methanol | - | - | - | - | - | - | | | | |
| | | | | | nur für MTA-Produktion | | | | | |
| Σ Summe | 21919,0 | 100,00 | 7500,0 | 100,00 | | | | | | |

# Tabelle IV

| Komponenten | 4.1 Filtrat A von 1. Umkristallisation kg/h | 4.1 Ma % | 4.2 Methanol-Brüden von Filtrat A Eindampfung kg/h | 4.2 Ma % | 4.3 Kristallbrei zur 1. Umkristallisation kg/h | 4.3 Ma % | 4.4 Isomeren zur Verbrennung kg/h | 4.4 Ma % | 4.5 Methanol-Brüden von Isomeren Eindampfung kg/h | 4.5 Ma % |
|---|---|---|---|---|---|---|---|---|---|---|
| DMT | 1219,4 | 2,05 | | | 1172,9 | 35,16 | 46,5 | 16,49 | | |
| DMI | 125,07 | 0,21 | | | 21,95 | 0,66 | 103,12 | 36,57 | | |
| DMO | 89,22 | 0,15 | | | 15,55 | 0,47 | 73,67 | 26,12 | | |
| HM-BME | 17,78 | 0,03 | | | 3,15 | 0,09 | 14,63 | 5,19 | | |
| TAE | 35,75 | 0,06 | | | 6,30 | 0,19 | 29,45 | 10,44 | | |
| p-TA | 17,78 | 0,03 | | | 3,15 | 0,09 | 14,63 | 5,19 | | |
| Methanol | 57986,0 | 97,47 | 54387,0 | 100,00 | 2113,0 | 63,34 | – | – | 1486,0 | 100,00 |
| Σ Summe | 59491,0 | 100,00 | 54387,0 | 100,00 | 3336,0 | 100,00 | 282,0 | 100,00 | 1486,0 | 100,00 |

# Tabelle V

| Komponenten | 5.1 DMT-Zwischenprodukt zur Hydrolyse | | 5.2 Destillat der Hydro-Methanol-Dest. | | 5.3 Demineralisiertes Wasser | | 5.4 Abwasser | | 5.5 PTA | |
|---|---|---|---|---|---|---|---|---|---|---|
| Analysen-Nr. | kg/h | Ma % | kg/h | Ma % | kg/h | Ma % | kg/h | Ma % | kg/h | Ma % |
| DMT | 21917,0 | 99,991 | | | | | | | | |
| DMI | 0,85 | 39 ppm | | | | | | | | |
| DMO | 0,61 | 28 ppm | | | | | | | | |
| HM-BME | 0,15 | 7 ppm | | | | | | | | |
| TAE | 0,24 | 11 ppm | | | | | | | | |
| p-TA | 0,15 | 7 ppm | | | | | 0,1 | 15 ppm | 0,05 | < 3 ppm |
| Methanol | | | 6927,0 | 51,84 | | | - | - | - | - |
| ITA | | | | | | | 0,53 | 78 ppm | 0,21 | 11 ppm |
| OTA | | | | | | | 0,37 | 54 ppm | 0,12 | 6 ppm |
| TA | | | | | | | 1,8 | 264 ppm | 18747,44 | 99,98 |
| TAS | | | | | | | - | - | 0,3 | 16 ppm |
| Wasser | | | 5995,0 | 44,87 | 17000,0 | 100,00 | 6799,30 | 99,87 | - | - |
| DME | | | 439,0 | 3,29 | - | - | - | - | - | - |
| MMT | | | - | - | - | - | 5,9 | 867 ppm | 1,88 | 100 ppm |
| Σ Summe | 21919,0 | 100,00 | 13361,0 | 100,00 | 17000,0 | 100,00 | 6808,0 | 100,00 | 18750,0 | 100,00 |

EP 0 464 046 B1

Tabelle VI

| Analysen-Nr. / Komponenten | 6.1 DMT-Zwischenprodukt zur Hydrolyse | | 6.2 Destillat der Hydro-Methanol-Dest. | | 6.3 Demineralisiertes Wasser | | 6.4 Abwasser | | 6.5 PTA | |
|---|---|---|---|---|---|---|---|---|---|---|
| | kg/h | Ma % | kg/h | Ma % | kg/h | Ma % | kg/h | Ma % | kg/h | Ma % |
| DMT | 21917,0 | 99,991 | | | | | | | | |
| DMI | 0,85 | 39 ppm | | | | | | | | |
| DMO | 0,61 | 28 ppm | | | | | | | | |
| HM-BME | 0,15 | 6 ppm | | | | | | | | |
| TAE | 0,24 | 11 ppm | | | | | | | | |
| p-TA | 0,15 | 6 ppm | | | | | 0128 | 18 ppm | 0,03 | 2 ppm |
| Methanol | | | 6927,0 | 51,84 | | | - | - | - | - |
| ITA | | | | | | | 0,59 | 87 ppm | 0,14 | 10 ppm |
| OTA | | | | | | | 0,38 | 46 ppm | 0,07 | 5 ppm |
| TA | | | | | | | 1,8 | 264 ppm | 14048,14 | 99,98 |
| TAS | | | | | | | - | - | 0,22 | 16 ppm |
| Wasser | | | 5995,0 | 44,87 | 17000,0 | 100,00 | 6799,21 | 99,87 | - | - |
| DME | | | 439,0 | 3,29 | - | - | - | - | - | - |
| MMT | | | - | - | - | - | 5,9 | 867 ppm | 1,4 | 100 ppm |
| Σ Summe | 21919,0 | 100,00 | 13361,0 | 100,00 | 17000,0 | 100,00 | 6808,0 | 100,00 | 14050,0 | 100,00 |

# Tabelle VI

| Analysen-Nr. | | | | | | | | |
|---|---|---|---|---|---|---|---|---|
| Komponenten | Ma % | kg/h | Ma % | kg/h | Ma % | kg/h | Ma % (6.6 PTA-p) | kg/h (6.6 PTA-p) |
| DMT | | | | | | | | |
| DMI | | | | | | | | |
| DMO | | | | | | | | |
| HM-BME | | | | | | | | |
| TAE | | | | | | | | |
| p-TA | | | | | | | 0,4 ppm | < 0,01 |
| Methanol | | | | | | | – | – |
| ITA | | | | | | | 2,4 ppm | < 0,01 |
| OTA | | | | | | | 1,4 ppm | < 0,01 |
| TA | | | | | | | 99,996 | 4699,8 |
| TAS | | | | | | | 16 ppm | 0,07 |
| Wasser | | | | | | | – | – |
| DME | | | | | | | – | – |
| MMT | | | | | | | 25 ppm | 0,11 |
| Σ Summe | | | | | | | 100,00 | 4700,0 |

## Patentansprüche

1. Verfahren zur Herstellung eines zur Weiterverarbeitung zu mittel- oder hochreinem Dimethylterephthalat (DMT) und/oder mittel- oder hochreiner Terephthalsäure (TA) bestimmten DMT-Zwischenproduktes mit

26

einem Gesamtanteil an Verunreinigungen von weniger als 1 Gew.-% und einem Gehalt an Terephthalaldehydsäuremethylester (TAE) von weniger als 100 ppm durch

a) gemeinsame Oxidation eines überwiegend para-Xylol (p-X) und para-Toluylsäuremethylester (p-TE) enthaltenden Gemisches in flüssiger Phase mit einem sauerstoffhaltigen Gas,

b) Veresterung der entstandenen Säuren mit Methanol,

c) destillative bzw. rektifikative Auftrennung der entstandenen Veresterungsprodukte in eine
   I) p-TE-reiche Fraktion,
   II) eine mehr als 99 Gew.-% DMT und dessen Isomere enthaltende Fraktion und
   III) eine schwersiedende Rückstandsfraktion,

d) Rückführung der p-TE-Fraktion I in die Oxidation a),

**dadurch gekennzeichnet,** daß die Rückstandsfraktion III einen DMT-Anteil von 15 bis 70 Gew.-% aufweist und daß

die DMT-Fraktion II durch einfache Lösungsmittel-Umkristallisation so weit zu dem DMT-Zwischenprodukt gereinigt wird, daß die Anteile an Hydroxymethyl-benzoesäuremethylester (HM-BME) und Terephthal aldehyd säure-methylester (TAE) zusammen weniger als 200 ppm im DMT-Zwischenprodukt betragen.

2. Verfahren nach Anspruch 1, **dadurch gekennzeichnet,** daß die Anteile an HM-BME und TAE in der DMT-Fraktion II zusammen weniger als 0,2 Gew.-% und bevorzugt zusammen weniger als 0,1 Gew.-% sowie die Anteile an HM-BME und TAE im DMT-Zwischenprodukt weniger als 50 ppm betragen.

3. Verfahren nach Anspruch 1 oder 2, **dadurch gekennzeichnet,** daß zusammen mit dem para-Xylol und der p-TE-Fraktion I zusätzlich 3 bis 7 Gew.-% DMT, bezogen auf die Summe aller der Oxidation a) in flüssiger Phase zugeführten Komponenten, der Oxidation a) zugeführt werden.

4. Verfahren nach Anspruch 3, **dadurch gekennzeichnet,** daß die der Oxidation zusätzlich zugeführte DMT-Menge durch Aufarbeitung der Rückstandsfraktion III gewonnen wird.

5. Verfahren nach einem der Ansprüche 1 bis 4, **dadurch gekennzeichnet,** daß zur rektifikativen Auftrennung der Veresterungsprodukte die Estermischung in einer ersten Rektifikationskolonne in ein die schwersiedende Rückstandsfraktion III bildendes Sumpfprodukt und ein Kopfprodukt getrennt wird, wobei der Druck in der Rektifikationskolonne am Sumpf 0,15 bis 0,5 bar und am Kopf 0,1 bis 0,4 bar und das Rücklaufverhältnis zwischen 0,2 und 1 beträgt, und daß das Kopfprodukt in einer zweiten Rektifikationskolonne in die DMT-Fraktion II als Sumpfprodukt und die p-TE-Fraktion I als Kopfprodukt aufgespalten wird, wobei der Druck in der zweiten Rektifikationskolonne am Sumpf 0,2 bis 0,7 bar und am Kopf 0,1 bis 0,5 bar und das Rücklaufverhältnis zwischen 0,5 und 2,5 beträgt.

6. Verwendung des nach einem der Ansprüche 1 bis 5 hergestellten DMT-Zwischenprodukts zur Herstellung von Reinst-DMT (DMT-p) durch eine zweite Lösungsmittel-Umkristallisation in Methanol.

7. Verwendung des nach einem der Ansprüche 1 bis 5 hergestellten DMT-Zwischenprodukts zur Herstellung von mittelreiner Terephthalsäure (MTA) durch Hydrolyse des DMT-Zwischenproduktes zu Terephthalsäure und Trennung der Terephthalsäure von der vorwiegend Wasser und Methanol enthaltenden Hydrolyse-Mutterlauge durch Auskristallisieren sowie Trocknung der Terephthalsäure (MTA).

8. Verwendung des nach einem der Ansprüche 1 bis 5 hergestellten DMT-Zwischenprodukts zur Herstellung von Terephthalsäure hoher Reinheit (PTA) durch Wäsche des DMT-Zwischenproduktes in Methanol, Hydrolyse des DMT zu Terephthalsäure und Trennung der Terephthalsäure von der vorwiegend Wasser und Methanol enthaltenden Hydrolyse-Mutterlauge durch Auskristallisieren sowie Trocknung der Terephthalsäure (PTA).

9. Verwendung des nach einem der Ansprüche 1 bis 5 hergestellten DMT-Zwischenproduktes zur Herstellung von Terephthalsäure höchster Reinheit mit einem Gehalt an Monomethylterephthalat (MMT) und para-Toluylsäure (p-TA) von zusammen kleiner 50 ppm (PTA-p) durch Wäsche des DMT-Zwischenproduktes in Methanol, Hydrolyse des DMT zu Terephthalsäure, Trennung der Terephthalsäure von der vorwiegend Wasser und Methanol enthaltenden Hydrolyse-Mutterlauge durch Auskristallisieren und anschließende Wäsche der auskristallisierten Terephthalsäure mit demineralisiertem Wasser sowie Trocknung der Terephthalsäure (PTA-p).

**10.** Verwendung des nach einem der Ansprüche 1 bis 5 hergestellten DMT-Zwischenproduktes zur gleichzeitigen Herstellung von Terephthalsäuren hoher und höchster Reinheit (PTA, PTA-p) durch Wäsche des DMT-Zwischenproduktes in Methanol, anschließende Hydrolyse des DMT zu Terephthalsäure, Auskristallisieren der Terephthalsäure, Trocknen eines Teils der Terephthalsäure und Ausschleusung als Terephthalsäure hoher Reinheit (PTA), Wäsche der weiteren auskristallisierten Terephthalsäure mit demineralisiertem Wasser und Trocknung zu Terephthalsäure höchster Reinheit mit einem Gesamtgehalt an Verunreinigungen einschließlich MMT und p-TA von kleiner 50 ppm (PTA-p) sowie Rückführung des Waschwassers in die Hydrolyse.

**Claims**

**1.** Process for the production of a DMT-intermediate product intended for working up to medium pure or pure dimethyl terephthalate (DMT-p) and/or medium pure or pure terephthalic acid (TA), the intermediate product having a total content of impurities of less than 1% by weight and a terephthalaldehydic acid methyl ester (TAE) content of less than 100 ppm by
    a) joint oxidation of a mixture containing predominantly para-xylene (p-X) and para-toluic acid methyl ester (p-TE) in the liquid phase with an oxygen-containing gas,
    b) esterification of the acids being produced with methanol,
    c) distillative or rectificative separation of the esterification products being produced into
        I) a p-TE rich fraction
        II) a fraction containing more than 99% by weight DMT and its isomers and
        III) a high boiling residue fraction
    d) feeding back of the p-TE fraction I to oxidation a),
characterised in that the residue fraction III has a DMT content of 15 to 70% by weight and that the DMT fraction II is purified by single solvent recrystallisation to the DMT intermediate product to such an extent that the amounts of hydroxymethylbenzoic acid methyl ester (HM-BME) and terephthalaldehydic acid methyl ester (TAE) amount together to less than 200 ppm in the DMT intermediate product.

**2.** Process according to claim 1, characterised in that the amounts of HM-BME and TAE in the DMT fraction II amount together to less than 0.2% by weight and preferably together to less than 0.1% by weight and in addition the amounts of HM-BME and TAE in the DMT intermediate product amount to less than 50 ppm

**3.** Process according to claim 1 or 2, characterised in that 3 to 7% by weight DMT, related to the sum of all components supplied to oxidation a) in the liquid phase, are supplied additionally to oxidation a) together with the para-xylene and the p-TE fraction I.

**4.** Process according to claim 3, characterised in that the amount of DMT supplied additionally to the oxidation is obtained by working up of residue fraction III.

**5.** Process according to one of claims 1 to 4, characterised in that, for the rectificative separation of the esterification products, the ester mixture is separated in a first rectification column into a sump product forming the high boiling residue fraction III and a head product, with the pressure in the rectification column amounting to 0.15 to 0.5 bar at the sump and 0.1 to 0.4 bar at the head and the reflux ratio amounting to between 0.2 and 1, and that the head product is split up in a second rectification column into the DMT fraction II as sump product and the p-TE fraction I as head product, with the pressure in the second rectification column amounting to 0.2 to 0.7 bar at the sump and to 0.1 to 0.5 bar at the head and the reflux ratio amounting to between 0.5 and 2.5.

**6.** Use of the DMT intermediate product produced according to one of claims 1 to 5 for the production of pure DMT (DMT-p) by a second solvent recrystallisation in methanol.

**7.** Use of the DMT intermediate product produced according to one of claims 1 to 5 for the production of medium pure terephthalic acid (MTA) by hydrolysis of the DMT intermediate product to form terephthalic acid and separation of the terephthalic acid from the hydrolysis mother liquor containing predominantly water and methanol by crystallising out as well as drying of the terephthalic acid (MTA).

8. Use of the DMT intermediate product produced according to one of claims 1 to 5 for the production of pure terephthalic acid (PTA) by washing of the DMT intermediate product in methanol, hydrolysis of the DMT to form terephthalic acid and separation of the terephthalic acid from the hydrolysis mother liquor containing predominantly water and methanol by crystallising out as well as drying of the terephthalic acid (PTA).

9. Use of the DMT intermediate product produced according to one of claims 1 to 5 for the production of terephthalic acid of very high purity with a monomethyl terephthalate (MMT) and para-toluic acid (p-TA) content of together less than 50 ppm (PTA-p) by washing of the DMT intermediate product in methanol, hydrolysis of the DMT to form terephthalic acid, separating of the terephthalic acid from the hydrolysis mother liquor containing predominantly water and methanol by crystallising out and then washing of the crystallised out terephthalic acid with deionised water as well as drying of the terephthalic acid (PTA-p).

10. Use of the DMT-intermediate product produced according to one of claims 1 to 5 for the simultaneous production of pure terephthalic acid and terephthalic acid of very high purity (PTA, PTA-p) by washing of the DMT intermediate product in methanol, then hydrolysis of the DMT to form terephthalic acid, crystallising out of the terephthalic acid, drying of part of the terephthalic acid and discharge as pure terephthalic acid (PTA), washing of the further crystallized out terephthalic acid with deionised water and drying to form terephthalic acid of very high purity with a total content of impurities including MMT and p-TA of less than 50 ppm (PTA-p), as well as recycling the washing water to the hydrolysis.

**Revendications**

1. Procédé de fabrication d'un produit intermédiaire de DMT destiné à être converti en téréphtalate de diméthyle (DMT) de pureté moyenne ou élevée et/ou en acide téréphtalique (TA) de pureté moyenne ou élevée, avec une fraction totale d'impuretés inférieure à 1 % en poids et une teneur en méthyl ester d'acide de téréphtalaldéhyde (TAE) de moins de 100 ppm par :
   a) oxydation conjointe d'un mélange contenant principalement du paraxylène (p-X) et du méthyl ester d'acide paratoluénique (p-TE) en phase liquide avec un gaz contenant de l'oxygène,
   b) estérification des acides produits par du méthanol,
   c) séparation par distillation ou rectification des produits d'estérification obtenus en
      I) une fraction riche en p-TE,
      II) une fraction contenant plus de 99 % en poids de DMT et de ses isomères, et
      III) une fraction de résidus lourds,
   d) recyclage de la fraction de p-TE I au stade d'oxydation a),
   caractérisé en ce que la fraction de résidus III présente une fraction de DMT de 15 à 70 % en poids et la fraction de DMT II est purifiée par simple recristallisation d'un solvant en produit intermediaire de DMT à un degré tel que les fractions de méthyl ester d'acide hydroxyméthylbenzoïque (HM-BME) et de méthylester d'acide de téréphtalaldéhyde (TAE) atteignent conjointement moins de 200 ppm dans le produit intermédiaire de DMT.

2. Procédé selon la revendication 1, caractérisé en ce que les fractions de HM-BME et de TAE dans la fraction de DMT II atteignent conjointement moins de 0,2 % en poids, de préférence, moins de 0,1 % en poids et les fractions de HM-BME et de TAE atteignent moins de 50 ppm dans le produit intermédiaire de DMT.

3. Procédé selon la revendication 1 ou 2, caractérisé en ce que, conjointement avec le paraxylène et la fraction de p-TE I, on ajoute également au stade d'oxydation a) 3 à 7 % en poids de DMT, par rapport à la somme de tous les composants ajoutés au stade d'oxydation a) en phase liquide.

4. Procédé selon la revendication 3, caractérisé en ce que la quantité de DMT acheminée en plus au stade d'oxydation est obtenue par traitement de la fraction de résidus III.

5. Procédé selon l'une quelconque des revendications 1 à 4, caractérisé en ce que, pour la séparation par rectification des produits d'estérification, le mélange d'esters est séparé dans une première colonne de rectification en un produit de bas de colonne formant la fraction de résidus lourds III et en un produit de tête, la pression dans la colonne de rectification étant sur le bas de colonne de 0,15 à 0,5 bar et sur la tête de colonne de 0,1 à 0,4 bar et le taux de reflux étant compris entre 0,2 et 1, et le produit de tête est scindé dans une deuxième colonne de rectification en fraction de DMT II comme produit de bas de colonne et en fraction de p-TE I comme produit de tête, la pression dans la deuxième colonne de rectification étant sur le bas de colonne de 0,2 à 0,7 bar et sur la tête de colonne de 0,1 à 0,5 bar et le taux de reflux étant compris entre 0,5 et 2,5.

6. Utilisation du produit intermédiaire de DMT fabriqué selon l'une des revendications 1 à 5 pour la fabrication de DMT très pur (DMT-p) par une deuxième recristallisation dans du méthanol comme solvant.

7. Utilisation du produit intermédiaire de DMT fabriqué selon l'une des revendications 1 à 5 pour la fabrication d'acide téréphtalique de pureté moyenne (MTA) par hydrolyse du produit intermédiaire de DMT en acide téréphtalique et séparation de l'acide téréphtalique de la lessive mère hydrolytique contenant principalement de l'eau et du méthanol par cristallisation ainsi que par séchage de l'acide téréphtalique (MTA).

8. Utilisation du produit intermédiaire de DMT fabriqué selon l'une des revendications 1 à 5 pour la fabrication d'acide téréphtalique de pureté élevée (PTA) par lavage du produit intermédiaire de DMT dans du méthanol, hydrolyse du DMT en acide téréphtalique et séparation de l'acide téréphtalique de la lessive mère hydrolytique contenant principalement de l'eau et du méthanol par cristallisation ainsi que par séchage de l'acide téréphtalique (PTA).

9. Utilisation du produit intermédiaire de DMT fabriqué selon l'une quelconque des revendications 1 à 5 pour la fabrication d'acide téréphtalique de très grande pureté avec une teneur conjointe en téréphtalate de monométhyle (MMT) et en acide paratoluénique (p-TA) inférieure à 50 ppm (PTA-p) par lavage du produit intermédiaire de DMT dans du méthanol, hydrolyse du DMT en acide téréphtalique, séparation de l'acide téréphtalique de la lessive hydrolytique contenant principalement de l'eau et du méthanol par cristallisation et ensuite lavage de l'acide téréphtalique cristallisé par de l'eau déminéralisée et séchage de l'acide téréphtalique (PTA-p).

10. Utilisation du produit intermédiaire de DMT fabriqué selon l'une quelconque des revendications 1 à 5 pour la préparation simultanée d'acides téréphtaliques de pureté élevée et très élevée (PTA, PTA-p) par lavage du produit intermédiaire du DMT dans du méthanol, ensuite hydrolyse du DMT en acide téréphtalique, cristallisation de l'acide téréphtalique, séchage d'une partie de l'acide téréphtalique que l'on sépare en tant qu'acide téréphtalique de pureté élevée (PTA), lavage de l'autre partie d'acide téréphtalique cristallisé à l'aide d'eau déminéralisée et séchage en acide téréphtalique de pureté très élevée avec une teneur totale en impuretés, y compris le MMT et le p-TA, de moins de 50 ppm (PTA-p) ainsi que recyclage de l'eau de lavage dans l'hydrolyse.

Fig. 1

EP 0 464 046 B1

Brüden

Brüden

Destillat
Destillat

Rückstand B

Rückstand A

Methanol

Methanolyse (Rückstand-Aufarbeitung)

Fig. 2

Fig. 3

EP 0 464 046 B1

**Filtrat-Aufbereitung und Isomeren-Ausschleusung**

Fig. 4

EP 0 464 046 B1

Destillat ⇦ (5.2)

DMT
Zwischenprodukt ⇨ (5.1)

Hydrolyse von DMT zu PTA

Fig. 5

EP 0 464 046 B1

⇨ PTA

⇨ Abwasser

⇦ Demin.
Wasser

Fig. 6

Hydrolyse von DMT zu PTA- and PTA-p